(19) Europäisches Patentamt
European Patent Office
Office européen des brevets

(11) EP 3 166 690 B2

(12) **NEUE EUROPÄISCHE PATENTSCHRIFT**
Nach dem Einspruchsverfahren

(45) Veröffentlichungstag und Bekanntmachung des Hinweises auf die Entscheidung über den Einspruch:
**08.09.2021 Patentblatt 2021/36**

(45) Hinweis auf die Patenterteilung:
**29.08.2018 Patentblatt 2018/35**

(21) Anmeldenummer: **15730107.8**

(22) Anmeldetag: **12.06.2015**

(51) Int Cl.:
***A61Q 5/08*** *(2006.01)* ***A61Q 5/10*** *(2006.01)*
***A61K 8/22*** *(2006.01)* ***A61K 8/23*** *(2006.01)*
***A61K 8/46*** *(2006.01)*

(86) Internationale Anmeldenummer:
**PCT/EP2015/063168**

(87) Internationale Veröffentlichungsnummer:
**WO 2016/005144 (14.01.2016 Gazette 2016/02)**

(54) **VERBESSERTE ENTFÄRBUNG VON GEFÄRBTEN KERATINISCHEN FASERN**
IMPROVED DECOLORISATION OF COLORED KERATINIC FIBERS
DÉCOLORATION AMÉLIORÉE DES FIBRES COLORÉS KERATINIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **09.07.2014 DE 102014213318**

(43) Veröffentlichungstag der Anmeldung:
**17.05.2017 Patentblatt 2017/20**

(73) Patentinhaber: **Henkel AG & Co. KGaA**
**40589 Düsseldorf (DE)**

(72) Erfinder:
- **SCHÖPGENS, Jürgen**
  **41366 Schwalmtal (DE)**
- **MÜLLER, Burkhard**
  **40221 Düsseldorf (DE)**

(56) Entgegenhaltungen:
**EP-A1- 1 470 812** **US-A- 5 264 001**
**US-A1- 2003 147 841** **US-A1- 2010 236 570**

- **"Colour B4 Hair Colour Remover", Mintel, April 2010 (2010-04), pages 1-5,**

Bemerkungen:
Die Akte enthält technische Angaben, die nach dem Eingang der Anmeldung eingereicht wurden und die nicht in dieser Patentschrift enthalten sind.

EP 3 166 690 B2

## Beschreibung

Die Erfindung betrifft Zusammensetzungen zum reduktiven Farbabzug von gefärbten keratinischen Fasern, insbesondere menschlichen Haaren, enthaltend in einem kosmetischen Träger mindestens ein Reduktionsmittel und mindestens ein zwitterionisches Tensid, das als Struktureinheiten jeweils eine quartäre Ammoniumgruppe und eine Gruppierung $-SO_3^-$ besitzt. Weiterhin sind auch eine Mehrkomponentenverpackungseinheit (Kit-of-parts) enthaltend die zuvor beschriebenen Zusammensetzungen und ein Verfahren zum Entfärben von Keratinfasern Gegenstand der Erfindung.

Zubereitungen zum Tönen und Färben von Haaren sind ein wichtiger Typ von kosmetischen Mitteln. Sie können dazu dienen, die natürliche Haarfarbe gemäß den Wünschen der entsprechenden Person leicht oder stärker zu nuancieren, eine gänzlich andere Haarfarbe zu erzielen oder unerwünschte Farbtöne, wie beispielsweise Grautöne, zu überdecken. Übliche Haarfärbemittel werden, je nach gewünschter Farbe bzw. Dauerhaftigkeit der Färbung, entweder auf Basis von Oxidationsfarbstoffen oder auf Basis von direktziehenden Farbstoffen formuliert. Häufig werden auch Kombinationen von Oxidationsfarbstoffen und direktziehenden Farbstoffen zur Erzielung spezieller Nuancen eingesetzt.

Färbemittel auf Basis von Oxidationsfarbstoffen führen zu brillanten und dauerhaften Farbtönen. Sie bedingen allerdings den Einsatz starker Oxidationsmittel, wie beispielsweise WasserstoffperoxidLösungen. Solche Färbemittel enthalten Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehrerer Kupplerkomponenten die eigentlichen Farbstoffe aus.

Färbemittel auf der Basis direktziehender Farbstoffe werden häufig für temporäre Färbungen eingesetzt. Bei den direktziehenden Farbstoffen handelt es sich um Farbstoffmoleküle, die direkt auf das Haar aufziehen und keinen oxidativen Prozess zur Ausbildung der Farbe benötigen. Wichtige Vertreter dieser Farbstoffklasse sind beispielsweise Triphenylmethanfarbstoffe, Azofarbstoffe, Anthrachinonfarbstoffe oder Nitrobenzolfarbstoffe, welche jeweils kationische oder anionische Gruppen tragen können.

Bei all diesen Färbeprozessen kann es vorkommen, dass die Färbung aus verschiedenen Gründen ganz oder teilweise wieder rückgängig gemacht werden soll. Eine teilweise Entfernung der Färbung bietet sich beispielsweise an, wenn das Färbeergebnis auf den Fasern dunkler ausfällt als gewünscht.

Andererseits kann auch eine vollständige Entfernung der Färbung in manchen Fällen gewünscht sein. So ist es beispielsweise denkbar, dass die Haare für einen konkreten Anlass in einer bestimmten Nuance gefärbt oder getönt werden sollen und nach einigen Tagen die ursprüngliche Farbe wieder zurückgewonnen werden soll.

Mittel und Verfahren zum Farbabzug sind bereits literaturbekannt. Ein aus dem Stand der Technik hinlänglich bekanntes Verfahren, Färbungen wieder rückgängig zu machen, ist die oxidative Nachbehandlung der gefärbten Haare, beispielsweise mithilfe eines üblichen Blondiermittels. Bei diesem Prozess können die Fasern aber durch den Einsatz starker Oxidationsmittel geschädigt werden.

Ferner wurden auch reduktive Prozesse zum Farbabzug bereits beschrieben. So offenbart beispielsweise die europäische Patentanmeldung EP 1 300 136 A2 Verfahren zur Haarbehandlung, bei welchem die Haare in einem ersten Schritt gefärbt und in einem zweiten Schritt wieder reduktiv entfärbt werden. Hierbei erfolgt die reduktive Entfärbung durch Anwendung von einer Formulierung enthaltend ein Dithionitsalz und ein Tensid. In der WO 2008/055756 A2 wird die reduktive Entfärbung von Keratinfasern mittels eines Gemisches aus einem Reduktionsmittel und einem Absorptionsmittel vorgenommen.

Bei Einsatz von reduktiven Entfärbemitteln findet die Entfärbung durch Reduktion der auf den Keratinfasern bzw. Haaren befindlichen Farbstoffe statt. Durch die Reduktion werden die Farbstoffe in der Regel in ihre reduzierten Leukoformen überführt. Bei diesem Vorgang werden die in den Farbstoffen vorhandenen Doppelbindungen reduziert, das chromophore System der Farbstoffe auf diese Weise unterbrochen und der Farbstoff in eine farblose Form umgewandelt.

Ein generelles Problem bei den aus dem Stand der Technik bekannten reduktiven Entfärbemitteln besteht darin, dass die gefärbten Keratinfasern durch Einsatz des Reduktionsmittels zwar zunächst entfärbt werden können, der Farbabzug jedoch nicht von Dauer ist. Insbesondere bei oxidativ gefärbten Haaren, bei denen die Färbung durch Oxidationsfarbstoffvorprodukte vom Entwickler- und vom Kupplertyp auf dem Haar erzeugt wird, werden Färbungen mit teilweise sehr guten Echtheitseigenschaften erhalten. Bei Anwendung des reduktiven Entfärbemittels werden diese Farbstoffe nun reduktiv in ungefärbte Verbindungen überführt - die aufgrund ähnlich guter Echtheitseigenschaften jedoch nach wie vor auf dem Haar verbleiben.

Nach Abspülen des Reduktionsmittels und unter Einwirkung von Luftsauerstoff können diese reduzierten Formen nun nach und nach wieder zurückoxidiert werden. Bedingt durch diese Rückoxidation findet eine mehr oder weniger stark ausgeprägte Rückfärbung statt. Diese Rückfärbung entspricht in der Regel nicht dem Farbton, in dem die Keratinfasern zuvor gefärbt worden waren, sondern kann beliebig unattraktiv ausfallen und wird vom Anwender des Entfärbemittels daher umso weniger erwünscht.

Aufgabe der vorliegenden Erfindung war daher die Bereitstellung eines Entfärbemittels zur Entfärbung von

gefärbten keratinischen Fasern, welches gefärbte Keratinfasern möglichst vollständig entfärbt. Die Entfärbung sollte lang anhaltend sein, und die entfärbten Keratinfasern sollten unter der Einwirkung von Luftsauerstoff keine Rückfärbung, keine Nuancenverschiebung und keine Nachdunklung erleiden. Das Entfärbemittel sollte insbesondere auf den Keratinfasern eine gute Entfärbeleistung zeigen, die zuvor mit oxidativen Färbemitteln auf Basis von Oxidationsfarbstoffvorprodukten vom Entwickler- und vom Kupplertyp gefärbt wurden.

Überraschenderweise hat sich nun herausgestellt, dass diese Aufgabe durch den Einsatz von Entfärbemitteln gelöst werden kann, die in einem wässrigen kosmetischen Träger ein oder mehrere anorganische Reduktionsmittel und mindestens ein spezielles zwitterionisches Tensid enthalten. Die speziellen zwitterionischen Tenside sind dadurch gekennzeichnet, dass die als Sturktureinheiten jeweils mindestens eine quartäre Ammoniumgruppe und eine Gruppierung -SO3- besitzen.

Ein erster Gegenstand der vorliegenden Erfindung ist ein Mittel zur reduktiven Entfärbung von gefärbten keratinischen Fasern, insbesondere menschlichen Haaren, enthaltend in einem wässrigen kosmetischen Träger

(a) ein oder mehrere Reduktionsmittel aus der Gruppe Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat, Ammoniumthiosulfat, Hydroxymethansulfinsäure, Aminomethansulfinsäure, Cystein, Thiomilchsäure, Thioglycolsäure und/oder Ascorbinsäure, und

(b) ein oder mehrere zwitterionische Tenside, die als Struktureinheiten jeweils mindestens eine quartäre Ammoniumgruppe und mindestens eine Gruppierung $-SO_3^-$ besitzen,

(c) ein oder mehrere Alkalisierungsmittel ausgewählt aus der Gruppe Natriumhydroxid. Kaliumhydroxid, Ammoniak. Monoethanolamin und/oder Arginin, dadurch gekennzeichnet, dass

- die Gesamtmenge aller im Mittel enthaltenen direktziehenden Farbstoffe und Oxidationsfarbstoffvorprodukte bei einem Wert von maximal 0.2 Gew.-% - bezogen auf das Gesamtgewicht des Mittels - liegt, und

- das Gewichtsverhältnis aus der Gesamtmenge aller im Mittel enthaltenen Reduktionsmittel aus der Gruppe (a) zu der Gesamtmenge aller im Mittel enthaltenen zwitterionischen Tenside aus der Gruppe (b), d.h. das Gewichtsverhältnis (a)/(b), bei einem Wert von mindestens 2,6 und von höchstens 7,0 liegt.

Die Anwendung der erfindungsgemäßen Entfärbemittel bewirkt - je nach Wahl der zuvor zur Färbung eingesetzten Farbstoffe - eine nahezu vollständige oder sogar vollständige Entfärbung von zuvor gefärbten Keratinfasern. Vor allem auf zuvor mit Oxidationsfarbstoffen gefärbten Keratinfasern zeigt das erfindungsgemäße Entfärbemittel eine hervorragende Wirkung. Als insbesondere überraschend hat sich herausgestellt, dass diese Entfärbewirkung auch nach dem Ausspülen des Reduktionsmittels noch anhält und dass auch die entfärbten Keratinfasern, die für Stunden bzw. Tage der Einwirkung von Luftsauerstoff ausgesetzt werden, keine Rückoxidation und keine Nachdunklung erleiden.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Entfärben von Keratinfasern bzw. menschlichen Haaren geeignet sind, steht prinzipiell einer Verwendung auch auf anderen Gebieten nichts entgegen.

Unter dem Begriff "gefärbte keratinische Fasern" werden Keratinfasern verstanden, die mit herkömmlichen, dem Fachmann bekannten kosmetischen Färbemitteln gefärbt wurden. Insbesondere snd unter den "gefärbten keratinsichen Fasern" Fasern zu verstehen, die mit den aus dem Stand der Technik bekannten oxidativen Färbemitteln und/oder mit direktziehenden Farbstoffen gefärbt wurden. Es wird in diesem Zusammenhang ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, die das entsprechende Wissen des Fachmannes wiedergeben.

Die Mittel enthalten die erfindungswesentlichen Inhaltsstoffe jeweils in einem wässrigen kosmetischen Träger, beispielsweise in einem geeigneten wässrigen oder wässrig-alkoholischen Träger. Zum Zwecke der reduktiven Entfärbung können solche Träger beispielsweise Cremes, Emulsionen, Gele oder auch tensidhaltige schäumende Lösungen, wie beispielsweise Shampoos, Schaumaerosole, Schaumformulierungen oder andere Zubereitungen, die für die Anwendung auf dem Haar geeignet sind, sein. Besonders bevorzugt handelt es sich bei Mitteln zum reduktiven Farbabzug von keratinischen Fasern um Cremes, Emulsionen oder fließfähige Gele.

Reduktionsmittel (a)

Als ersten erfindungswesentlichen Inhaltsstoff (a) enthalten die Entfärbemittel ein oder mehrere Reduktionsmittel aus der Gruppe Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat, Ammoniumthiosulfat, Natriumdisulfit, Kaliumdisulfit, Ammoniumdisulfit, Hydroxymethansulfinsäure, Aminomethansulfinsäure, Cystein, Thiomilchsäure, Thioglycolsäure (Alternativname: Sulfanylessigsäure) und/oder Ascorbinsäure.

Bei Natriumdithionit handelt es sich um ein anorganischen Reduktionsmittel mit der Summenformel $Na_2S_2O_4$ und der CAS-Nr. 7775-14-6.

Bei Zinkdithionit handelt es sich um ein anorganisches Reduktionsmittel mit der Summenformel $ZnS_2O_4$ und der CAS-Nr. 7779-86-4.

Bei Kaliumdithionit handelt es sich um ein anorganisches Reduktionsmittel mit der Summenformel $K_2S_2O_4$ und der CAS-Nr. 14293-73-3.

Bei Natriumsulfit handelt es sich um ein anorganisches Reduktionsmittel mit der Summenformel $Na_2SO_3$ und der CAS-Nr. 7757-83-7.

Bei Natriumhydrogensulfit handelt es sich um ein anorganisches Reduktionsmittel mit der Summenformel $NaHSO_3$ und der CAS-Nr. 7631-90-5. Natriumhydrogensulfit wird bevorzugt in Form einer wässrigen Lösung eingesetzt.

Bei Kaliumsulfit handelt es sich um ein ein anorganisches Reduktionsmittel mit der Summenformel $K_2SO_3$ und der CAS-Nr. 10117-38-1.

Bei Kaliumhydrogensulfit handelt es sich um ein anorganisches Reduktionsmittel mit der Summenformel $KHSO_3$ und der CAS-Nr. 7773-03-7.

Bei Ammoniumsulfit handelt es sich um ein anorganisches Reduktionsmittel mit der Summenformel $(NH_4)_2SO_3$ und der CAS-Nr. 10196-04-0.

Bei Natriumthiosulfat handelt es sich um ein anorganisches Reduktionsmittel mit der Summenformel $Na_2S_2O_3$ und der CAS-Nr. 7772-98-7.

Bei Kaliumthiosulfat handelt es sich um ein anorganisches Reduktionsmittel mit der Summenformel $K_2S_2O_3$ und der CAS-Nr. 10294-66-3.

Bei Ammoniumthiosulfat handelt es sich um ein anorganisches Reduktionsmittel mit der Summenformel $(NH_4)_2S_2O_3$ und der CAS-Nr. 7783-18-8.

Bei Hydroxymethansulfinsäure handelt es sich um ein organisches Reduktionsmittel mit der Formel HO-$CH_2$-S(O)OH und der CAS-Nr. 79-25-4. Alternativ wird Hydroxymethansulfinsäure auch als Formaldehydsulfoxylsäure bezeichnet. Erfindungsgemäß ist sowohl der Einsatz der Hydroxymethansulfinsäure selbst als auch der Einsatz der physiologisch verträglichen Salze von Hydroxymethansulfinsäure, beispielsweise des Natriumsalzes und/oder des Zinksalzes. Der Einsatz von Natriumformaldehydsulfoxylat (Natrium Hydroxymethansulfinat, dem Natriumsalz der Hydroxymethansulfinsäure) und/oder Zinkformeladehydsulfoxylat (Zink Hydroxymethansulfinat, dem Zinksalz der Hydroxymethansulfinsäure) ist demnach ebenfalls erfindungsgemäß.

Bei Aminomethansulfinsäure handelt es sich um ein organisches Reduktionsmittel mit der Formel $H_2N$-$CH_2$-S(O)OH und der CAS-Nr. 118201-33-5. Erfindungsgemäß ist sowohl der Einsatz der Aminomethansulfinsäure selbst als auch der Einsatz der physiologisch verträglichen Salze von Aminomethansulfinsäure, beispielsweise des Natriumsalzes und/oder des Zinksalzes. Der Einsatz von Natrium Aminomethansulfinat (dem Natriumsalz der Aminomethansulfinsäure) und/oder Zink Aminomethansulfinat (dem Zinksalz der Aminomethansulfinsäure) ist deshalb ebenfalls erfindungsgemäß.

Unter Cystein (2-Amino-3-sulfanylpropionsäure) wird erfindungsgemäß D-Cystein, L-Cystein und/oder ein Gemisch aus D- und L-Cystein verstanden.

Unter Thiomilchsäure (2-Sulfanylpropionsäure) wird D-Thio-Milchsäure, L-Thio-Milchsäure und/oder ein Gemisch aus D- und L-Thiomilchsäure verstanden. Erfindungsgemäß ist sowohl der Einsatz der Thiomilchsäure selbst als auch der Einsatz von Thiomilchsäure in Form eines physiologisch verträglichen Salzes hiervon. Ein bevorzugtes Salz der Thiomilchsäure ist Ammoniumthiolactat. Bei Ammoniumthiolactat handelt es sich um das Ammoniumsalz der Thiomilchsäure (d.h. das Ammoniumsalz der 2-Sulfanylpropionsäure) (Formel XX).

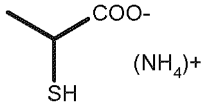

(Formel XX)

Von der Definition Ammoniumthiolactat mit umfasst sind sowohl die Ammoniumsalze der D-Thiomilchsäure als auch die Ammoniumsalze der L-Thiomilchsäure sowie deren Gemische.

Unter Thioglycolsäure (Sulfanylessigsäure, 2-Mercapto-essigäure) wird ein organisches Reduktionsmittel der Formel HS-$CH_2$-COOH verstanden, die Verbindung besitzt die CAS-Nr. 68-11-1. Auch bei Thioglycolsäure ist sowohl der Einsatz von Thioglycolsäure selbst auch der Einsatz eines physiologisch verträglichen Salzes der Thioglycolsäure erfindungsgemäß. Als physiologisch verträgliche Salze der Thiolgycolsäure können beispielsweis Natriumthioglycolat, Kaliumthioglycolat und/oder Ammoniumthioglycolat eingsetzt werden. Ammoniumthioglylat ist ein bevorzugtes physiologisch vertägliches Salze der Thioglycolsäure.

Bei Ammoniumthioglycolat handelt es sich um das Ammoniumsalz der Thiglycolsäure (d.h. das Ammoniumsalz

der Sulfanylessigsäure) (Formel XXX).

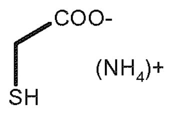

(Formel XXX)

Unter Ascorbinsäure wird erfindungsgemäß insbesondere (R)-5-[(S)-1,2-Dihydroxyethyl]-3,4-dihydroxy- 5*H*-furan-2-on (weitere Alterniativnamen: Vitamin C, L-Ascorbinsäure) mit der CAS-Nr. 50-81-7 verstanden.

Als besonders gut geeignet zur reduktiven Entfärbung von oxidativ gefärbten Haaren haben sich die Reduktionsmittel Natriumdithionit, Zinkdithionit, Kaliumdithionit, Natriumsulfit, Natriumhydrogensulfit, Kaliumsulfit, Kaliumhydrogensulit, Ammoniumsulfit, Natriumthiosulfat, Kaliumthiosulfat und/oder Ammoniumthiosulfat herausgestellt. Werden diese vorgenannten bevorzugten Reduktionsmittel (a) in Kombination mit den erfindungsgemäßen zwitterionischen Tensiden (b) eingesetzt, so konnte eine besonders effektive Entfärbung und eine besonders wirksame Verhinderung der Rückoxidation bei den entfärbten Haarsträhnen beobachtet werden. Auf diese Weise konnte ein Nachdunkeln der entfärbten Keratinfasern über einen besonders langen Zeitraum verhindert werden.

Weiterhin werden das bzw. die Reduktionsmittel aus der Gruppe (a) bevorzugt in bestimmten Mengenbereichen eingesetzt. Ein entfärbender Effekt ist bereits bei kleinen Einsatzmengen zu beobachten. Um eine ausreichende und starke Entfärbewirkung zu erhalten, ist es jedoch bevozugt, wenn das Entfärbemittel das oder die Reduktionsmittel (a) in einer Gesamtmenge von 0,5 bis 20,5 Gew.-%, bevorzugt von 3,5 bis 15,5 Gew.-%, weiter bevorzugt von 6,0 bis 13,5 Gew.-% und besonders bevorzugt von 7,5 bis 11,5 Gew.-% - bezogen auf das Gesamtgewicht des Mittels - enthält.

Bevorzugt ist daher ein Mittel zur reduktiven Entfärbung von gefärbten keratinischen Fasern, insbesondere menschlichen Haaren, enthaltend in einem wässrigen kosmetischen Träger

(a) ein oder mehrere Reduktionsmittel aus der Gruppe Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat, Ammoniumthiosulfat, Hydroxymethansulfinsäure, Aminomethansulfinsäure, Cystein, Thiomilchsäure, Thioglycolsäure und/oder Ascorbinsäure in einer Gesamtmenge von 0,5 bis 20,5 Gew.-%, bevorzugt von 3,5 bis 15,5 Gew.-%, weiter bevorzugt von 6,0 bis 13,5 Gew.-% und besonders bevorzugt von 7,5 bis 11,5 Gew.-% - bezogen auf das Gesamtgewicht des Mittels - und

(b) ein oder mehrere zwitterionische Tenside, die als Struktureinheiten jeweils mindestens eine quartäre Ammoniumgruppe und eine Gruppierung $-SO_3^-$ besitzen.

Ein ganz besonders bevorzugtes Mittel zur reduktiven Entfärbung von gefärbten keratinischen Fasern ist weiterhin dadurch gekennzeichnet, dass es

(a) ein oder mehrere Reduktionsmittel aus der Gruppe Natriumdithionit, Zinkdithionit, Kaliumdithionit, Natriumsulfit, Natriumhydrogensulfit, Kaliumsulfit, Kaliumhydrogensulfit, Ammoniumsulfit,Natriumthiosulfat, Kaliumthiosulfat und/oder Ammoniumthiosulfat in einer Gesamtmenge von 0,5 bis 20,5 Gew.-%, bevorzugt von 3,5 bis 15,5 Gew.-%, weiter bevorzugt von 6,0 bis 13,5 Gew.-% und besonders bevorzugt von 7,5 bis 11,5 Gew.-% - bezogen auf das Gesamtgewicht des Mittels - enthält.

Weiterhin hat es sich als besonders vorteilhaft herausgestellt, wenn die erfindungsgemäßen Mittel bestimmte Kombinationen aus Reduktionsmitteln aus der Gruppe (a) enthalten, da bei bestimmten Kombinationen ein ganz besonders starker entfärbender Effekt auftritt. Besonders vorteilhaft ist in diesem Zusammenhang der Einsatz von zwei verschiedenen Reduktionsmitteln aus der Gruppe (a), wobei das Entfärbemittel

(a1) ein erstes Reduktionsmittel enthält, das aus der Gruppe Natriumdithionit, Zinkdithionit, Kaliumdithionit, Natriumthiosulfat, Kaliumthiosulfat und/oder Ammoniumthiosulfat ausgewählt wird, und zusätzlich

(a2) ein zweites Reduktionsmittel enthält, das aus der Gruppe Natriumsulfit, Natriumhydrogensulfit, Kaliumsulfit, Kaliumhydrogensulfit und/oder Ammoniumsulfit ausgewählt wird.

Mit anderen Worten besonders bevorzugt sind im Rahmen dieser Ausführungsform die Mittel zur reduktiven Entfärbung von gefärbten keratinischen Fasern, insbesondere menschlichen Haaren, die in einem wässrigen kosmetischen Träger enthalten

(a1) ein erstes Reduktionsmittel, das aus der Gruppe Natriumdithionit, Zinkdithionit, Kaliumdithionit, Natriumthiosulfat, Kaliumthiosulfat und/oder Ammoniumthiosulfat ausgewählt wird, und zusätzlich

(a2) ein zweites Reduktionsmittel, das aus der Gruppe Natriumsulfit, Natriumhydrogensulfit, Kaliumsulfit, Kaliumhydrogensulfit und/oder Ammoniumsulfit ausgewählt wird, und

(b) ein oder mehrere zwitterionische Tenside, die als Struktureinheiten jeweils mindestens eine quartäre Ammoniumgruppe und eine Gruppierung $-SO_3^-$ besitzen.

Zwitterionische Tenside (b)

Als zweiten erfindungswesentlichen Bestandteil (b) enthalten die Entfärbemittel ein oder mehrere zwitterionische Tenside, die als Struktureinheiten jeweils mindestens eine quartäre Ammoniumgruppe und mindestens eine Gruppierung $-SO_3^-$ besitzen.

Unter Tensiden werden amphiphile (bifunktionelle) Verbindungen mit mindestens einem hydrophoben Rest und mindestens einem hydrophilen Molekülteil verstanden. Bei dem hydrophoben Molekülteil handelt es sich zumeist um eine Kohlenwasserstoffkette mit 10 bis 30 Kohlenstoff-Atomen.

Im Fall der zwitterionischen Tenside umfasst der hydrophile Molekülteil eine zwitterionische Struktureinheit, d.h. eine Struktureinheit, die sowohl einen kationisch geladenen als auch einen anionisch geladenen Molekülteil umfasst. Erfindungsgemäße zwitterionische Tenside (b) sind dadurch gekennzeichnet, dass sie einen kationisch geladenen Molekülteil in Form einer quartären Ammoniumgruppe besitzen und ihr anionischer Molekülteil in Form einer Gruppierung $-SO_3^-$ vorliegt.

Eine Ammoniumgruppe ist quartär, wenn eine Gruppierung des Typs $(R_1R_2R_3R_4N)^+$ vorliegt, d.h. wenn alle vier H-Atome des $NH_4$-Ions, von dem die quartäre Ammoniumgruppe abgeleitet ist, durch organische Reste R (bzw. R1 bis R4) ersetzt wird.

Die Gruppierung $-SO3^-$ des zwitterionischen Tensids kann direkt an ein Kohlenstoffatom gebunden vorliegen. In diesem Fall handelt es sich bei dem anionischen Teil der zwitterionischen Verbindung um eine deprotonierte Sulfonsäuregruppierung der Formel $R_5R_6R_7C-SO3^-$; die Reste R5, R6, R7 stellen hierbei den restlichen Teil des erfindungsgemäßen zwitterionischen Tensids dar.

Es ist jedoch ebenfalls auch erfindungsgemäß, wenn die Gruppierung $-SO_3^-$ über ein Sauerstoffatom an ein Kohlenstoffatom gebunden vorliegt, in diesem Fall stellt der anionische Teil des zwitterionischen Tensids einen deprotonierten Schwefelsäureester der Formel $R_5R_6R_7C-O-SO_3^-$ dar, wobei die Reste R5, R6, R7 wieder den restlichen Teil des erfindungsgemäßen zwitterionischen Tensids darstellen.

Erfindungsgemäß bevorzugt ist es, wenn die Gruppierung $-SO3^-$ direkt an ein Kohlenstoffatom gebunden, d.h. in Form einer deprotonierten Sulfonsäuregruppierung, vorliegt.

Über einen besonders langen Zeitraum ließ sich eine Nachdunklung der entfärbten Keratinfasern verhindern, wenn als erfindungsgmäßes zwitterionisches Tensid (b) mindestens eine Verbindung der Formel (I) eingesetzt wurde,

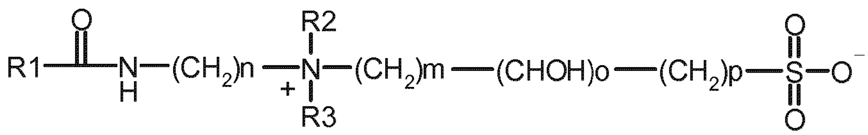

(I)

wobei

R1 für eine lineare oder verzweigte $C_9$-$C_{29}$-Alkylgruppe, eine lineare oder verzweigte $C_9$-$C_{29}$-Alkenylgruppe oder eine lineare oder verzweigte Hydroxy-$C_9$-$C_{29}$-Alkylgruppe steht,

R2, R3 unabhängig voneinander für eine $C_1$-$C_6$-Alkylgruppe, eine $C_2$-$C_6$-Alkenylgruppe oder eine Hydroxy$C_2$-$C_6$-alkylgruppe stehen,

n für eine ganze Zahl von 1 bis 6 steht,

m für eine ganze Zahl von 0 bis 6 steht,

o für eine ganze Zahl von 0 bis 6 steht,

p für eine ganze Zahl von 0 bis 6 steht,

mit der Maßgabe, das die Summe aus m, o und p mindestens 1 beträgt.

In einer weiteren Ausführungsform ganz besonders bevorzugt ist daher ein Mittel zur reduktiven Entfärbung von gefärbten keratinischen Fasern, enthaltend

(a) ein oder mehrere Reduktionsmittel aus der Gruppe Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat und/oder Ammoniumthiosulfat und

(b) ein oder mehrere zwitterionisches Tenside der Formel (I),

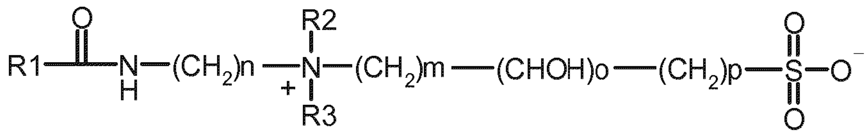

**(I)**

worin

R1 für eine lineare oder verzweigte $C_9$-$C_{29}$-Alkylgruppe, eine lineare oder verzweigte $C_9$-$C_{29}$-Alkenylgruppe oder eine lineare oder verzweigte Hydroxy-$C_9$-$C_{29}$-Alkylgruppe steht,
R2, R3 unabhängig voneinander für eine $C_1$-$C_6$-Alkylgruppe, eine $C_2$-$C_6$-Alkenylgruppe oder eine Hydroxy-$C_2$-$C_6$-alkylgruppe stehen,
n für eine ganze Zahl von 1 bis 6 steht,
m für eine ganze Zahl von 0 bis 6 steht,
o für eine ganze Zahl von 0 bis 6 steht,
p für eine ganze Zahl von 0 bis 6 steht,

mit der Maßgabe, das die Summe aus m, o und p mindestens 1 beträgt.

Der Einsatz der erfindungsgemäßen zwitterionischen Tenside (b) verhindert die Nachdunklung der entfärbten Strähnen und sorgt auf diese Weise für einen lang anhaltenden Entfärbeeffekt. Von besonderem Vorteil ist hierbei der Einsatz der Tenside in bestimmten Mengenbereichen. Besonders bevorzugt ist es, wann das oder die zwitterionischen-Tenside (b) im erfindungsgemäßen Entfärbemittel in einer Gesamtmenge von von 0,1 bis 15,0 Gew.-%, bevorzugt von 0,5 bis 12,0 Gew.-%, weiter bevorzugt von 1,0 bis 9,0 Gew.-% und besonders bevorzugt von 2,0 bis 6,0 Gew.-% - bezogen auf das Gesamtgewicht des Mittels - eingesetzt werden.

In einer weiteren ganz besonders bevorzugten Ausführungsform ist ein erfindungsgmäßes Entfärbemittel daher dadurch gekennzeichnet, dass es als zwitterionisches Tensid (b) ein oder mehrere Tenside der Formel (I) in einer Gesamtmenge von 0,1 bis 15,0 Gew.-%, bevorzugt von 0,5 bis 12,0 Gew.-%, weiter bevorzugt von 1,0 bis 9,0 Gew.-% und besonders bevorzugt von 2,0 bis 6,0 Gew.-% - bezogen auf das Gesamtgewicht des Mittels - enthält,

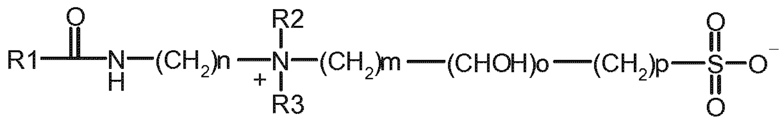

(I)

worin

R1 für eine lineare oder verzweigte $C_9$-$C_{29}$-Alkylgruppe, eine lineare oder verzweigte $C_9$-$C_{29}$-Alkenylgruppe oder eine lineare oder verzweigte Hydroxy-$C_9$-$C_{29}$-Alkylgruppe steht,
R2, R3 unabhängig voneinander für eine $C_1$-$C_6$-Alkylgruppe, eine $C_2$-$C_6$-Alkenylgruppe oder eine Hydroxy$C_2$-$C_6$-alkylgruppe stehen,
n für eine ganze Zahl von 1 bis 6 steht,
m für eine ganze Zahl von 0 bis 6 steht,
o für eine ganze Zahl von 0 bis 6 steht,
p für eine ganze Zahl von 0 bis 6 steht,

mit der Maßgabe, das die Summe aus m, o und p mindestens 1 beträgt.

Insbesondere bevorzugt ist der Einsatz von zwitterionischen Tensiden der Formel (I), bei welchen der Rest R1 für eine lineare $C_{11}$-Alkylgruppe, eine lineare $C_{13}$-Alkylgruppe, eine lineare $C_{15}$-Alkylgruppe, eine lineare $C_{17}$-Alkylgruppe oder eine lineare $C_{19}$-Alkylgruppe, eine lineare, einfach ungesättigte $C_{11}$-Alkenylgruppe, eine lineare, einfach ungesättigte $C_{13}$-Alkenylgruppe, eine lineare, einfach ungesättigte $C_{17}$-Alkenylgruppe oder eine lineare, einfach ungesättigte $C_{19}$-Alkenylgruppe steht.

Weiterhin ist es ganz besonders bevorzugt, wenn n für die Zahl 3 steht.

Die Reste R2 und R3 stehen bevorzugt unabhängig voneinander für eine $C_1$-$C_6$-Alkylgruppe, besonders bevorzugt stehen R2 und R3 für eine Methylgruppe.

m steht ganz besonders bevorzugt für die Zahlen 1 oder 2.
o steht ganz besonders bevorzugt für die Zahl 1.
p steht ganz besonders bevorzugt für die Zahlen 1 oder 2.

In einer weiteren ganz besonders bevorzugten Ausführungsform ist ein erfindungsgemäßes Entfärbemittel deshalb dadurch gekennzeichnet, dass es als zwitterionisches Tensid (b) ein oder mehrere Tenside der Formel (I) in einer Gesamtmenge von 0,1 bis 15,0 Gew.-%, bevorzugt von 0,5 bis 12,0 Gew.-%, weiter bevorzugt von 1,0 bis 9,0 Gew.-% und besonders bevorzugt von 2,0 bis 6,0 Gew.-% - bezogen auf das Gesamtgewicht des Mittels - enthält,

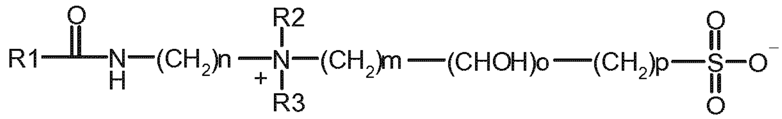
(I)

worin

| | |
|---|---|
| R1 | für eine lineare $C_{11}$-Alkylgruppe, eine lineare $C_{13}$-Alkylgruppe, eine lineare $C_{15}$-Alkylgruppe, eine lineare $C_{17}$-Alkylgruppe, eine lineare $C_{19}$-Alkylgruppe, eine lineare, einfach ungesättigte $C_{11}$-Alkenylgruppe, eine lineare, einfach ungesättigte $C_{13}$-Alkenylgruppe, eine lineare, einfach ungesättigte $C_{17}$-Alkenylgruppe oder eine lineare, einfach ungesättigte $C_{19}$-Alkenylgruppe steht, |
| R2, R3 | beide für eine Methylgruppe stehen, |
| n | für eine ganze Zahl von 1 bis 3 steht, |
| m | für die Zahl 1 oder 2 steht |
| o | für die Zahl 1 steht und |
| p | für die Zahl 1 oder 2 steht. |

Die zwitterionischen Tenside dieses ganz besonders bevorzugten Typs sind auch unter dem Namen "Amidopropylhydroxysultaine" bekannt:
Laurylamidpropylhydroxysultain ist ein zwitterionischesTensid der Formel (I), wobei R1 für C11-Alkyl, R2 für Methyl, R3 für Methyl, n für 3, m für 1, o für 1 und p für 1 steht.

Cocamidopropylhydroxysultain stellt ein Gemisch von Verbindungen der Formel (I) dar, bei denen R1 steht für C11-Alkyl bis C17-Alkyl, R2 für Methyl, R3 für Methyl, n für 3, m für 1, o für 1 und p für 1.

Oleamidopropylhydroxysultain ist ein zwitterionisches Tensid der Formel (I), wobei R1 für eine einfach ungesättigte C17-Alkenylgruppe steht (die Doppelbindung liegt zwischen den Atomen 8 und 9 des R1-Restes), R2 für Methyl, R3 für Methyl, n für 3, m für 1, o für 1 und p für 1 steht.

Explizit ganz besonders bevorzugt wird Cocamidopropyl Hydroxysultain eingesetzt, wie es beispielsweise unter dem Handelsnamen Mirataine CBS von der Firma Rhodia käuflich erhältlich ist. Ganz besonders bevorzugt wird erfindungsgemäß demnach ein Gemisch von zwitterionische Tensiden (b) der Formel (I) eingesetzt, bei denen R1 steht für C11-Alkyl bis C17-Alkyl, R2 für Methyl, R3 für Methyl, n für 3, m für 1, o für 1 und p für 1.

Das oder die zwitterionischen Tenside (b) werden zusammen mit dem Reduktionsmittel (a) in einem wässrigen kosmetischen Träger eingesetzt und bilden so das erfindungsgemäße Entfärbemittel. Dieses Entfärbemittel kann durch Zusatz von Säuren und Basen auf verschiedene pH-Werte eingestellt werden. Die Gruppierung $-SO3^-$ der Tenside (b) kann in dem wässrigen kosmetischen Träger bei Veränderung des pH-Wertes in einer Gleichgewichtsreaktion auch teilweise protoniert werden. Auch diese durch die Gleichgewichtsreaktion protonierten Formen der zwitterinionischen Tenside sind von dieser Erfindung mit umfasst. Alle angegebenen Mengenangaben beziehen sich auf die Gesamtmenge der zwitterionischen Tenside, die in ihrer zwitterionischen Form dem wässrigen Träger hinzugefügt werden.

Mengenverhältnis (a)/(b)

Das bzw. die Reduktionsmittel (a) und das bzw. die zwitterionischen Tenside (b) bewirken zusammen eine effektive, lang anhaltende Entfärbung von gefärbten Keratinfasern. Hierbei bewirkt der Zusatz der speziellen zwitterionischen Tenside mit $-SO3^-$ Gruppierung, dass nach Abschluss des Entfärbevorgangs unter dem Einfluss von Luftsauerstoff keine Rückoxidation stattfindet. Auf diese Weise kann ein Nachdunkeln und damit eine spätere Abschwächung des Entfärbeergebnisses verhindert werden. Da der gesamte Entfärbeprozess somit eine Wechselwirkung der Reduktionsmittel (a) und der zwitterionischen Tenside (b) erfordert, werden die Wirkstoffe beider Kategorien (a) und (b) in bestimmten Verhältnisssen zueinander eingesetzt.

Es hat sich herausgestellt, dass die Nachdunklung besonders effektiv verhindert werden kann, wenn die Reduktionsmittel (a) im Vergleich zu den zwitterionischen Tensiden (b) mindestens in gleicher Gesamtmenge, bevorzugt jedoch in einem Überschuß eingesetzt werden. Die Gesamtmenge der Reduktionsmittel (a) wird um den Faktor 2,6 und besonders bevorzugt um den Faktor 3,2 höher als die Gesamtmenge der zwitterionischen Tenside (b) gewählt.

Die Entfärbemittel sind daher dadurch gekennzeichnet, dass das Gewichtsverhältnis aus der Gesamtmenge aller im Mittel enthaltenen Reduktionsmittel aus der Gruppe (a) zu der Gesamtmenge aller im Mittel enthaltenen zwitterionischen Tenside aus der Gruppe (b), d.h. das Gewichtsverhältnis (a)/(b), bei einem Wert von mindestens 2,6 und besonders bevorzugt von mindestens 3,2 liegt.

Beispiel:

100 g Entfärbemittel enthalten 8,0 g Natriumdithionit (Gesamtmenge aller Reduktionsmittel (a)). Bei Einsatz von 8,0 g Cocamidopropylhydroxysultain (Aktivsubstanz) liegt das Gewichttsverhältnis (a)/(b) bei einem Wert von 1,0. Bei Einsatz von 4,4 g Cocamidopropylhydroxysultain liegt das Gewichtsverhältnis (a)/(b) bei einem Wert von ca. 1,8. Bei Einsatz von 3,1 g Cocamidopropylhydroxysultain liegt das Gewichtsverhältnis (a)/(b) bei einem Wert ca. 2,6. Bei Einsatz von 2,5 g Cocamidopropylhydroxysultain liegt das Gewichtsverähltnis (a)/(b) bei einem Wert ca. 3.2.

Auch wenn sich der Einsatz eines Gewichtsüberschusses an Reduktionsmittel (a) als vorteilhaft herausgestellt hat, so sollte die Einsatzmenge an zwitterionischen Tensiden (b) auch nicht zu klein gewählt werden, damit die Nachdunklung der entfärbten Strähnen in ausreichendem Maß inhibiert werden kann. Weiterhin erfindungsgemäß ist es daher, dass die Gesamtmenge der im Mittel enthaltenen Reduktionsmittel (a) die Gesamtmenge der zwitterionischen Tenside (b) höchstens um den Faktor 7,0, bevorzugt höchstens um den Faktor 6,2 weiter bevorzugt höchstens um den Faktor 5,6 und besonders bevorzugt höchstens um den Faktor 4,8 übersteigt.

Die Entfärbemittel sind daher dadurch gekennzeichnet, dass das Gewichtsverhältnis aus der Gesamtmenge aller im Mittel enthaltenen Reduktionsmittel aus der Gruppe (a) zu der Gesamtmenge aller im Mittel enthaltenen zwitterionischen Tenside aus der Gruppe (b), d.h. das Gewichtsverhältnis (a)/(b), bei einem Wert von höchstens 7,0, bevorzugt von höchstens 6,2, weiter bevorzugt von höchstens 5,6 und besonders bevorzugt von höchstens 4,8 liegt.

Beispiel:

100 g Entfärbemittel enthalten 8,0 g Natriumditionit (Gesamtmenge aller Reduktionsmittel (a)).

Bei Einsatz von 1,14 g Cocamidopropylhydroxysultain liegt das Gewichttsverhältnis (a)/(b) bei einem Wert von ca. 7,0. Bei Einsatz von 1,29 g Cocamidopropylhydroxysultain liegt das Gewichtsverhältnis (a)/(b) bei einem Wert von ca. 6,2. Bei Einsatz von 1,42 g Cocamidopropylhydroxysultain liegt das Gewichtsverhältnis (a)/(b) bei einem Wert ca. 5,6. Bei Einsatz von 1,66 g Cocamidopropylhydroxysultain liegt das Gewichtsverähltnis (a)/(b) bei einem Wert ca. 4,8.

Zusammenfassend ist es am allermeisten bevorzugt, wenn das Gewichtsverhältnis aus der Gesamtmenge aller im Mittel enthaltenen Reduktionsmittel aus der Gruppe (a) zu der Gesamtmenge aller im Mittel enthaltenen zwitterionischen Tenside aus der Gruppe (b), d.h. das Gewichtsverhältnis (a)/(b), bei einem Wert von 3,2 bis 4,8 liegt.

Polvole

Es hat sich herausgestellt, dass der Einsatz von Polyolen die Entfärbewirkung weiter unterstützt. Aus diesem Grund ist es bevorugt, wenn die erfindungsgemäßen Entärbemittel zusätzlich ein odere mehrere Polyole enthalten.

Unter einem Polyol wird eine Verbindung mit mindestens zwei aliphatischen (d.h. nicht phenolischen) OH-Gruppen verstanden.

Beispiele für geeignete erfindungsgemäße Polyole sind insbesondere Ethylengylcol, 1,2-Propylenglycol, 1,3-Propandiol, 1,2-Butandiol, 1,3-Butandiol, 1,2-Pentandiol, 1,3-Pentandiol, 1,4-Pentandiol, 1,5-Pentandiol, 1,2-Hexandiol, 1,3-hexandiol, 1,4-Hexandiol, 1,5-Hexandiol und 1,6-Hexandiol. Geeignet sind aber auch Polyethylenglycol und Polypropylenglycol.

In einer weiteren bevorzugten Ausführungsform ist ein erfindungsgemäßes Entfärbemittel daher dadurch gekennzeichnet, dass es zusätzlich ein oder mehrere Polyole aus der Gruppe Ethylengylcol (1,2-Ethandiol), 1,2-Propandiol, 1,3-Propandiol, 1,2-Butandiol, 1,3-Butandiol, 1,4-Butandiol, 1,2-Pentantiol, 1,3-Pentantiol, 1,4-Pentantiol, 1,5-Pentantiol, 1,2-Hexandiol, 1,3-Hexandiol, 1,4-Hexantiol, 1,5-Hexandiol, 1,6-Hexandiol, Polyethylenglycol und/oder Polypropylenglycol enthält.

Die Polyole werden in den erfindungsgemäßen Mitteln bevorzugt in einer Gesamtmenge von 0,5 bis 15,0 Gew.-%, bevorzugt von 2,5 bis 13,5 Gew.-%, weiter bevorzugt von 3,5 bis 11,5 Gew.-% und besonders bevorzugt von 4,5 bis 9,5 Gew.-% - bezogen auf das Gesamtgewicht des Mittels - eingesetzt.

In einer weiteren bevorzugten Ausführungsform ist ein erfindungsgemäßes Entfärbemittel daher dadurch gekennzeichnet, dass es zusätzlich ein oder mehrere Polyole in einer Gesamtmenge von 0,5 bis 15,0 Gew.-%, bevorzugt von 2,5 bis 13,5 Gew.-%, weiter bevorzugt von 3,5 bis 11,5 Gew.-% und besonders bevorzugt von 4,5 bis 9,5 Gew.-%

- bezogen auf das Gesamtgewicht des Mittels - enthält.

Weitere Tenside

Die erfindungsgemäßen Entfärbemittel können neben den erfindungswesentlichen zwitterionischen Tensiden (b) auch noch weitere Tenside enthalten, wie beispielsweise nichtionische, ampholytische und/oder kationische Tenside. Bevorzugt ist es jedoch, wenn die Entfärbemittel keine anionischen Tenside enthalten.

In einer weiteren bevorzugten Ausführungsform ist ein erfindungsgemäßes Entfärbemittel daher dadurch gekennzeichnet, dass die Gesamtmenge aller im Mittel enthaltenen anionischen Tenside bei einem Wert von maximal 0,5 Gew.-%, bevorzugt von maximal 0,3 Gew.-%, weiter bevorzugt von maximal 0,1 Gew.-% und besonders bevorzugt von maximal 0,05 Gew.-% - bezogen auf das Gesamtgewicht des Mittels - liegt.

Unter anionischen Tensiden im Sinne dieser Erfindung werden Tenside mit ausschließlich anionischer Ladung verstanden.

Demnach ist es bevorzugt, wenn die Gesamtmenge aller im Mittel enthaltenen anionischen Tenside aus der Gruppe der

- linearen Fettsäuren mit 10 bis 22 C-Atomen (Seifen),
- Ethercarbonsäuren der Formel R-O-(CH2-CH2O)x-CH2-COOH, in der R eine lineare Alkylgruppe mit 10 bis 22 C-Atomen und x = 0 oder 1 bis 16 ist,
- Acylsarcosiden mit 10 bis 18 C-Atomen in der Acylgruppe,
- Acyltauriden mit 10 bis 18 C-Atomen in der Acylgruppe,
- Acylisethionaten mit 10 bis 18 C-Atomen in der Acylgruppe,
- Sulfobernsteinsäuremono- und -dialkylestern mit 8 bis 18 C-Atomen in der Alkylgruppe und Sulfobernsteinsäuremono-alkylpolyoxyethylestern mit 8 bis 18 C-Atomen in der Alkylgruppe und 1 bis 6 Oxyethylgruppen,
- linearen Alkansulfonaten mit 12 bis 18 C-Atomen,
- linearen Alpha-Olefinsulfonaten mit 12 bis 18 C-Atomen,
- Alpha-Sulfofettsäuremethylestern von Fettsäuren mit 12 bis 18 C-Atomen, und der
- Alkylsulfaten und Alkylpolyglykolethersulfaten der Formel R-O(CH2-CH2O)x-SO3H, in der R eine bevorzugt lineare Alkylgruppe mit 10 bis 18 C-Atomen und x = 0 oder 1 bis 12 ist,

bei einem Wert von maximal 0,5 Gew.-%, bevorzugt von maximal 0,3 Gew.-%, weiter bevorzugt von maximal 0,1 Gew.-% und besonders bevorzugt von maximal 0,05 Gew.-% - bezogen auf das Gesamtgewicht des Mittels - liegt.

Unter nichtionischen Tensiden werden amphiphile (bifunktionelle) Verbindungen mit mindestens einem hydrophoben Rest und mindestens einem hydrophilen Molekülteil verstanden. Bei dem hydrophoben Molekülteil handelt es sich zumeist um eine Kohlenwasserstoffkette mit 10 bis 30 Kohlenstoff-Atomen. Als hydrophile Gruppe tragen die nichtionischen Tenside beispielsweise eine Polyolgruppe, eine Polyalkylenglykolethergruppe oder eine Kombination aus Polyol- und Polyglykolethergruppe. Solche Verbindungen sind beispielsweise Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in derAlkylgruppe, C12-C22-Fettsäuremono- und -diester von Anlagerungsprodukten von 1 bis 30 Mol Ethylenoxid an Glycerin, C8-C22-Alkylmono- und -oligoglycoside und deren ethoxylierte Analoga sowie Anlagerungsprodukte von 5 bis 60 Mol Ethylenoxid an Rizinusöl und gehärtetes Rizinusöl. Bevorzugte nichtionische Tenside sind Alkylpolyglykoside.

Die zusätzlich einsetzbaren nichtionischen Tenside können in den erfindungsgemäßen Entfärbemitteln in einer Gesamtmenge von 0,1 bis 15,0 Gew.-%, bevorzugt von 2,5 bis 13,5 Gew.-%, weiter bevorzugt von 3,5 bis 11,5 Gew.-% und besonders bevorzugt von 4,5 bis 9,5 Gew.-% - bezogen auf das Gesamtgewicht des Mittels - eingesetzt werden.

Erfindungsgemäß werden als kationische Tenside insbesondere solche vom Typ der quartären Ammoniumverbindungen, der Esterquats und der Amidoamine eingesetzt. Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der

Bezeichnung Tegoamid® S 18 im Handel erhältliche Stearamidopropyldimethylamin dar.

Weitere erfindungsgemäß verwendbare kationische Tenside stellen die quaternisierten Proteinhydrolysate dar.

Die zusätzlich einsetzbaren kationischen Tenside können in den erifndungsgemäßen Entfärbemitteln in einer Gesamtmenge von 0,1 bis 15,0 Gew.-%, bevorzugt von 2,5 bis 13,5 Gew.-%, weiter bevorzugt von 3,5 bis 11,5 Gew.-% und besonders bevorzugt von 4,5 bis 9,5 Gew.-% - bezogen auf das Gesamtgewicht des Mittels - eingesetzt werden.

Entfärbung gefärbter Keratinfasern

Bei den erfindungsgemäßen Mitteln handelt es sich um Entfärbemittel, die zur Entfärbung von zuvor gefärbten keratinischen Fasern, insbesondere menschlichen Haaren eingesetzt werden. Bei den gefärbten Keratinfasern handelt es sich üblicherweise um Fasern, die zuvor mit herkömmlichen, dem Fachmann bekannten Oxidationsfarbstoffen und/oder direktziehenden Farbstoffen gefärbt wurden. Die Entfärbemittel sind geeignet zur Entfernung von Färbungen, die mit Oxidationsfarbstoffen auf Basis von Entwickler- und Kupplerkomonenten auf den Keratinfasern erzeugt wurden. Wenn als Entwickler die folgenden Verbindungen einsetzt wurden, lassen sich die hiermit erzeugten Färbungen durch Einsatz des Entfärbemittel gut, effektiv und nahezu ohne spätere Nachdunklung entfernen: p-Phenylendiamin, p-ToluylendiaminN,N-Bis-(β-hydroxyethyl)-p-phenylendiamin, 4-N,N-Bis-(β-hydroxyethyl)-amino-2-methylanilin, 2-(β-Hydroxyethyl)-p-phenylendiamin, 2-(α,β-Dihydroxyethyl)-p-phenylendiamin, 2-Hydroxymethyl-p-phenylendiamin, Bis-(2-hydroxy-5-aminophenyl)-methan, p-Aminophenol, 4-Amino-3-methylphenol, 2,4,5,6-Tetraaminopyrimidin, 4-Hydroxy-2,5,6-triaminopyrimidin, 2-Hydroxy-4,5,6-triaminopyrimidin und/oder 4,5-Diamino-1-(β-hydroxyethyl)-pyrazol.

Wenn als Kuppler die folgenden Verbindungen einsetzt wurden, lassen sich die hiermit erzeugten Färbungen ebenfalls mit sehr gutem Entfärbeergebnis entfernen: m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

1-Naphthol, 1,5-Dihydroxynaphthalin, 2,7-Dihydroxynaphthalin, 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Das zu entfärbende Substrat kann ebenso mit direktziehenden Farbstoffen eingefärbt worden sein. Als direktziehende Farbstoffe kommen dabei insbesondere Nitrophenylendiamine, Nitroaminophenole, Azofarbstoffe, Anthrachinone oder Indophenole in Frage. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Weiterhin können die zu entfärbenden Substrate auch mit in der Natur vorkommenden, natürlichen Farbstoffen, wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, gefärbt sein.

Die erfindungsgmäßen Entfärbemittel sind zur Entfernung dieser Färbungen gedacht und enhalten selbst daher bevorzugt keine Farbstoffe, d.h. keine Oxidationsfarbstoffvorprodukte vom Entwicklertyp und vom Kupplertyp und auch keine direktziehenden Farbstoffe.

Ein erfindungsgemäßes Entfärbemittel ist daher dadurch gekennzeichnet, dass die Gesamtmenge aller im Mittel enthaltenen direktziehenden Farbstoffe und Oxidationsfarbstoffvorprodukte bei einem Wert von maximal 0,2 Gew.-%, bevorzugt von maximal 0,1 Gew.-%, weiter bevorzugt von maximal 0,05 Gew.-% und besonders bevorzugt von maximal 0,01 Gew.-% - bezogen auf das Gesamtgewicht des Mittels - liegt.

pH-Wert und Alkalisierungsmittel

Bei dem zuvor beschriebenen Entfärbemitteln handelt es sich um Mittel, welche die erfindungswesentlichen Komponenten (a) und (b) in einem wässrigen kosmetischen Träger enthalten. Der pH-Wert der Entfärbemittel kann prinzipiell auf Werte zwischen 2 bis 12 eingestellt werden. Aus Stabilitätsgründen und zum Zwecke der Lagerung ist es

jedoch von Vorteil, wenn das Mittel einen alkalischen pH-Wert besitzt. Bevorzugt wird das Mittel daher auf einen pH-Wert von 7 bis 12, weiter bevorzugt von 7,5 bis 11,5 noch weiter bevorzugt von 8,0 bis 11,0 und ganz besonders bevorzugt von 8,5 bis 10,5 eingestellt.

Die pH-Werte wurden mit einer Glaselektrode des Typs N 61 der Firma Schott bei einer Temperatur von 22 °C gemessen.

In einer weiteren bevorzugten Ausführungsform ist ein erfindungsgemäßes Entfärbemittel daher dadurch gekennzeichnet, dass es einen pH-Wert von 7,0 bis 12,0, bevorzugt von 7,5 bis 11,5, weiter bevorzugt von 8,0 bis 11,0 und ganz besonders bevorzugt von 8,5 bis 10,5 (gemessen mit einer Glaselektrode des Typs N61 der Firma Schott bei einer Temperatur von 22 °C) besitzt.

Die erfindungsgemäßen Entfärbemittel werden durch Einsatz eines oder mehrere Alkalisierungsmittel aus der Gruppe Natriumhydroxid, Kaliumhydroxid, Ammoniak, Monoethanolamin und/oder Arginin auf einen alkalischen pH-Wert gebracht. Ganz besonders bevorzugt werden aus dieser Gruppe Natriumhydroxid und/oder Kaliumhydroxid ausgewählt.

Ein erfindungsgemäßes Entfärbemittel ist daher dadurch gekennzeichnet, dass es zusätzlich ein oder mehrere Alkalisierungsmittel ausgewählt aus der Gruppe Natriumhydroxid, Kaliumhydroxid, Ammoniak, Monoethanolamin und/oder Arginin, besonders bevorzugt ausgewählt aus der Gruppe Natriumhydroxid und/oder Kaliumhydroxid, enthält.

Gele

Abhängig von den zuvor für die Färbung verwendeten Farbstoffen und abhängig vom Zustand, dem Schädigungsgrad und der Dicke der Haare kann der Entfärbeprozess bei unterschiedlichen Personen unterschiedlich lange dauern. Auch wenn der Entfärbeprozess generell innerhalb der üblichen Anwendungszeiten von bis zu 60 Minuten abgeschlossen sein sollte, ist es für den Anwender des Entfärbemittels doch um ein vielfaches komfortabler, den Entfärbevorgang direkt zu beobachten und das Entfärbemittel direkt nach Abschluss der Entfärbung - gegebenenfalls bereits nach 20 oder 30 Minuten - abspülen zu können.

Die direkte Beobachtung des Entfärbeprozesses wird für den Anwender oder Friseur möglich, wenn das Entfärbemittel in Form eines transparenten Gels konfektioniert wird.

In einer weiteren bevorzugten Ausführungsform ist ein erfindungsgemäßes Entfärbemittel daher dadurch gekennzeichnet, dass es sich um eine klare, fließfähige Gelformulierung mit einem Transmissionsgrad T von mindestens 70 %, bevorzugt von mindestens 75 %, weiter bevorzugt von mindestens 80 % und besonders bevorzugt mindestens 85 % handelt, wobei sich der Transmissionsgrad T über die folgende Formel berechnet

$$T = \Phi ex/\Phi in$$

mit

- $\phi$ex gleich der Strahlungsintensität des aus dem Mittel austretenden, durchgelassenen Lichtstrahls und
- $\phi$in gleich der Strahlungsintensität des in das Mittel einfallenden Lichtstrahls.

Unter Gelen werden Systeme verstanden, die aus einem festen, kolloidisch verteilten Stoff, bei dem es sich um ein Verdickungsmittel bzw. einem Geliermittel handelt, und einer Flüssigkeit (Wasser oder Wasser-Lösungsmittel-Gemische) verstanden. Hierbei bildet das Verdickungs- bzw. Geliermittel in der Flüssigkeit ein räumliches Netwerk.

Die Gele im Sinne der vorliegenden Erfindung sind fließfähig, was bedeutet, dass sie bevorzugt eine Viskosität von 1.000 mPas bis 15.000 mPas, besonders bevorzugt von 3.000 mPas bis 8.000 mPas (bei Messungen mit einem Rotationsviskosimeter von Brookfield, Spindelgröße 4, bei 25°C und 20 Upm) besitzen.

Die Transmission beschreibt die Durchlässigkeit des Gels für das Messlicht (bevorzugt Tageslicht), und wird als Transmissionsgrad T ausgedrückt. Der Transmissionsgrad stellt einen Verhältniswert T = $\Phi$ex/$\Phi$in dar, wobei die Strahlungsintensität des aus dem Mittel austretenden, durchgelassenen Lichtstrahls ($\Phi$ex) zur Strahlungsintensität des in das Mittel einfallenden Lichtstrahls ($\Phi$in) ins Verhältnis gesetzt wird. Die Messung erfolgt mit Tageslicht (Tageslichtlampe) bei einer Schichtdicke von 1 cm (d.h. das zu vermessende Gel wird in eine Küvette eingefüllt, so dass das Gel in einer Schichtdicke von 1 cm vorliegt und wird dann mit einem handelsüblichen Photometer vermessen). Bei einem Transmissionsgrad von mindestens 70%, bevorzugt von mindestens 75 %, weiter bevorzugt von mindestens 80 % und besonders bevorzugt mindestens 85 % ist das Gel so transparent, dass der Konsument den Entfärbeprozess des Haares direkt durch das transparente, auf das Haar aufgetragene Gel hindurch beobachten kann. Auf diese Weise kann er die Beendigung des Entfärbeprozesses direkt optisch wahrnehmen, ohne des Entfärbemittel von einer Probesträhne des behandelten Haares abspülen zu müssen.

Als Geliermittel bzw. Verdickungsmittel zur Herstellung des transparenten Gels können beispielsweise Agar-Agar, Carrageen, Alginate, Xanthan-Gum, Karaya-Gummi, Ghatti-Gummi, Tragant, Skleroglucangums oder Gummi arabicum, Alginate, Pektine, Polyosen, Guar-Gums, Johannisbrotbaumkernmehl, Leinsamengummen, Dextrane, Pektine, Staerke-Fraktionen und Derivate wie Amylose, Amylopektin und Dextrine, Gelatine und Casein sowie Cellulosederivate, wie beispielsweise Methylcellulose, Carboxyalkylcellulosen wie Carboxymethylcellulose und Hydroxyalkylcellulosen wie Hydroxyethylcellulose eingsetzt werden.

Bei Einsatz von Xanthan können die Entfärbungsmittel in Form von fließfähigen Gelen konfektioniert werden, deren Viskosität im Hinblick auf den Anwendungsprozess optimiert ist und die besonders gut eine direkte visuelle Beurteilung des Entfärbevorgangs ermöglichen. Aus diesem Grund ist der Einsatz von Xanthanals Verdickungsmittel besonders bevorzugt.

In einer weiteren bevorzugten Ausführungsform ist ein erfindungsgemäßes Entfärbemittel daher dadurch gekennzeichnet, dass es zusätzlich als Verdickungsmittel Xanthan enthält.

Kit-of-Parts

Wie zuvor beschrieben wird das Entfärbemittel zum Zwecke der Lagerung bevorzugt auf einen alkalischen pH-Wert eingestellt, da es bei alkalischen pH-Werten eine bessere Stabiltität besitzt. Die Entfärbewirkung der Kombination aus Reduktionsmittel (a) und zwitterionischem Tensid (b) ist jedoch eine Funktion des pH-Wertes, die ihr Optimium im sauren Milieu durchläuft. Aus diesem Grund ist es von Vorteil, das erfindungsgemäße Mittel kurz vor der Anwendung auf einen sauren pH-Wert zu bringen, der bevorzugt im Bereich von 4 bis 6 liegt. Kurz vor der Anwendung sollte der pH-Wert des zuvor alkalisch eingestellten Mittels daher abgesenkt werden.

Die Ansäuerung des zuvor alkalisch eingestellten Entfärbemittels kurz vor der Anwendung kann durch Vermischen von zwei verschiedenen Mitteln realisiert werden, wobei das zuvor beschriebene alkalisch eingestellte erste Mittel mit einem weiteren Mittel vermischt wird, welches eine oder mehrere Säuren enthält.

Als Säuren können beispielsweise eine oder mehrere Säuren aus der Gruppe Zitronensäure, Weinsäure, Äpfelsäure, Milchsäure, Essigsäure, Schwefelsäure, Salzsäure, Phosphorsäure, Methansulfonsäure, Benzoesäure Malonsäure, Oxalsäure und/oder 1-Hydroxyethan-1,1-diphosphonsäure verwendet werden.

Ein zweiter Gegenstand der vorliegenden Erfindung ist eine Mehrkomponentenverpackungseinheit (Kit-of-parts) zur reduktiven Entfärbung von gefärbten keratinischen Fasern, welche getrennt voneinander konfektioniert

(I) in einem Container (I) ein Mittel (A) und
(II) in einem Container (II) ein Mittel (B) umfasst, wobei

- das Mittel (A) in Container (I) ein Mittel des ersten Erfindungsgegenstands ist
- das Mittel (B) in Container (II) ein kosmetisches Mittel ist, das mindestens eine organische und/oder anorganische Säure aus der Gruppe Zitronensäure, Weinsäure, Äpfelsäure, Milchsäure, Essigsäure, Schwefelsäure, Salzsäure, Phosphorsäure, Methansulfonsäure, Benzoesäure Malonsäure, Oxalsäure und/oder 1-Hydroxyethan-1,1-diphosphonsäure enthält.

Mit anderen Worten wird das anwendungsbereite Entfärbemittel durch Vermischen des Mittels (A) aus Container (I) und des Mittels (B) aus Container (II) hergestellt.

Bei dem Mittel (A) des ersten Erfindungsgegentstand handelt es sich um das - bevorzugt alkalisch eingestellte und lagerstabile - Mittel, welches das bzw. die Reduktionsmittel (a) sowie das bzw. die zwitterionischen Tenside (b) enthält. Durch Vermischen des Mittels (A) mit dem ein oder mehrere Säuren enthaltenden Mittel (B) wird der pH-Wert des anwendungsbereiten Mittels abgesenkt und auf den für die Anwendung optimalen Wert gebracht.

Als besonders geeignet zur Einstellung des pH-Wertes haben sich die Säuren Zitronensäure, Weinsäure, Äpfelsäure, Milchsäure, Methansulfonsäure, Malonsäure, Oxalsäure und/oder 1-Hydroxyethan-1,1-diphosphonsäure erwiesen.

Die Mittel (B) in Container (II), welche mindestens eine Säure enthalten, können in fester oder flüssiger Form konfektioniert werden. Bevorzugt handelt es sich bei dem Mittel (B) in Container (II) um ein flüssiges Mittel mit einem wässrigen Träger.

Das Mittel (B) in Container (II) besitzt bevorzugt einen pH-Wert von 1 bis 6, bevorzugt von 1,3 bis 4,5, weiter bevorzugt von 1,6 bis 4,0 und besonders bevorzugt von 2,0 bis 3,6, wobei diese pH-Werte beispielsweise mit einer Glaselektrode des Typs N61 der Firma Schott bei einer Tempteratur von 22 °C gemessen werden können.

Besonders bevorzugt ist demnach eine Mehrkomponentenverpackungseinheit (Kit-of-parts), wobei

- das Mittel (B) in Container (II) ein Mittel ist, das in einem wässrigen kosmetischen Träger mindestens eine organische Säure aus der Gruppe Zitronensäure, Weinsäure, Äpfelsäure, Milchsäure, Methansulfonsäure, Malonsäure, Oxalsäure und/oder 1-Hydroxyethan-1,1-diphosphonsäure enthält und einen pH-Wert von 1 bis 6, bevorzugt von 1,3 bis

4,5, weiter bevorzugt von 1,6 bis 4,0 und besonders bevorzugt von 2,0 bis 3,6 (gemessen mit einer Glaselektrode des Typs N61 der Firma Schott bei einer Tempteratur von 22 °C) besitzt.
Gegebenenfalls kann die Mehrkomponentenverpackungseinheit (Kit-of-parts) auch noch einen weiteren, dritten getrennt konfektionierten Container (III) umfassen, wobei der Container (III) ein Mittel (C) enthält, bei dem es sich um ein Shampoo oder ein Kontitioniermittel handeln kann. Ebenfalls bevorzgut ist daher auch eine Mehrkomponentenverpackungseinheit (Kit-of-parts) zur reduktiven Entfärbung von gefärbten keratinischen Fasern, welche getrennt voneinander konfektioniert

(I) in einem Container (I) ein Mittel (A)
(II) in einem Container (II) ein Mittel (B),
(III) in einem Container (III) ein Mittel (C) umfasst, wobei

- das Mittel (A) in Container (I) ein Mittel des ersten Erfindungsgegenstands ist
- das Mittel (B) in Container (II) ein kosmetisches Mittel ist, das mindestens eine organische und/oder anorganische Säure aus der Gruppe Zitronensäure, Weinsäure, Äpfelsäure, Milchsäure, Essigsäure, Schwefelsäure, Salzsäure, Phosphorsäure, Methansulfonsäure, Benzoesäure Malonsäure, Oxalsäure und/oder 1-Hydroxyethan-1,1-diphosphonsäure enthält,
- das Mittel (C) in Container (III) ein kosmetisches Mittel ist, das ein oder mehrere zwitterionische Tenside enthält. Ebenfalls bevorzugt ist weiterhin auch eine Mehrkomponentenverpackungseinheit (Kit-of-parts) zur reduktiven Entfärbung von gefärbten keratinischen Fasern, welche getrennt voneinander konfektioniert

(I) in einem Container (I) ein Mittel (A)
(II) in einem Container (II) ein Mittel (B),
(III) in einem Container (III) ein Mittel (C) umfasst, wobei

- das Mittel (A) in Container (I) ein Mittel des ersten Erfindungsgegenstands ist
- das Mittel (B) in Container (II) ein kosmetisches Mittel ist, das mindestens eine organische und/oder anorganische Säure aus der Gruppe Zitronensäure, Weinsäure, Äpfelsäure, Milchsäure, Essigsäure, Schwefelsäure, Salzsäure, Phosphorsäure, Methansulfonsäure, Benzoesäure Malonsäure, Oxalsäure und/oder 1-Hydroxyethan-1,1-diphosphonsäure enthält,
- das Mittel (C) in Container (III) ein kosmetisches Mittel ist, das ein oder mehrere zwitterionische Tenside enthält, die als Struktureinheiten jeweils mindestens eine quartäre Ammoniumgruppe und eine Gruppierung $-SO_3^-$ besitzen.

Mischungsverhältnis der Mittel (A) und (B)

Wie bereits zuvor beschrieben wird das anwendungsbereite Entfärbemittel bevorzugt durch Vermischen der Mittel (A) und (B) hergestellt. Prinzipiell können die Mittel (A) und (B) hierbei in verschiedenen Mischungsverhältnissen, wie beispielsweise (A)/(B) von 20:1 bis 1:20, vermischt werden.
Um eine komfortable Vermischung zu gewährleisten, kann von Vorteil sein, die beiden Mittel (A) und (B) in ungefähr gleichen Mengen einzusetzen.
Insbesondere, wenn die im Mittel (B) enthaltenen Säuren in konzentrierterer Form eingesetzt werden, kann es jedoch auch von Vorteil sein, das Mittel (A) in einem Überschuss einzusetzen.
In einer weiteren bevorzugten Ausführungsform ist eine erfindungsgemäße MehrkomponentenVerpackungseinheit daher dadurch gekennzeichnet, dass die Mengen des Mittels (A) in Container (I) und des Mittels (B) in Container (II) so gewählt werden, dass bei Herstellung der Anwendungsmischung - d.h. bei Vermischung der Mittel (A) und (B) - das Mischungsverhältnis (A)/(B) bei einem Wert von mindestens 1, bevorzugt von mindestens 1,3, weiter bevorzugt von mindestens 1,5 und besonders bevorzugt von mindestens 2,0 liegt.
Zur Herstellung der Mischung kann beispielsweise das Mittel (A) aus Container (I) vollständig in Container (II) - der bereits das Mittel (B) enthält - überführt werden. In diesem Fall wird die Größe des Containers (II) so gewählt, dass der Container (II) die Gesamtmenge der Mittel (A) und (B) aufnehmen kann und auch ein Vermischen der beiden Mittel (A) und (B), z.B. durch Verschütteln oder Verrühren, zulässt.
Analog kann die Herstellung der Mischung auch durch vollständige Übeführung des Mittels (B) aus Container (II) in Container (I) - der bereits das Mittel (A) enthält - erfolgen. In diesem Fall sollte die Größe des Containers (I) so gewählt werden, dass der Container (I) die Gesamtmenge der Mittel (A) und (B) aufnehmen kann und auch ein Vermischen der beiden Mittel (A) und (B), z.B. durch Verschütteln, oder Verrühren, zulässt.
Eine weitere Möglichkeit zur Herstellung der Anwendungsmischung ist die vollständige Überführung beider Mittel (A) und (B) aus den Containern (I) und (II) in ein drittes Behältnis, welches dann das Vermischen beider Mittel - z.B. durch Verschütteln, oder Verrühren - erlaubt.

Beispiel: Eine erfindungsgemäße Mehrkompenenten-Verpackungseinheit enthält

- 100 g des Mittels (A) in Container (I)
- 50 g des Mittels (B) in Conatiner (II)

Zur Herstellung der Anwendungsmischung wird das Mittel (B) aus Container (II) vollständig in Container (I) überführt. Die Mittel (A) und (B) werden dann miteinander verschüttelt oder verrührt. Das Mischungsverhältnis der Mittel (A)/(B) liegt bei einem Wert von (100 g / 50 g) = 2,0.

Weitere Inhaltsstoffe

Die Mittel (A), (B) sowie gegegenenfalls (C) können weiterhin zusätzliche Wirk-, Hilfs- und Zusatzstoffe enthalten, um die Entfärbeleistung zu verbessern und weitere gewünschte Eigenschaften der Mittel einzustellen. Beispielsweise kann eines oder mehrere der Mittel zusätzlich nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylamino-ethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, $N_2O$, Dimethylether, $CO_2$ und Luft. Es wird in diesem Zusammenhang ausdrücklich auf die bekannten Monographien, z. B. Kh. Schrader, Grundlagen und Rezepturen der Kosmetika, 2. Auflage, Hüthig Buch Verlag, Heidelberg, 1989, verwiesen, die das entsprechende Wissen des Fachmannes wiedergeben.

Verfahren

Die erfindungsgemäßen, zuvor beschriebenen Mittel und Mehrkomponenten-Verpackungseinheiten (Kit-of-Parts) lassen sich in Verfahren zur Färbung und reduktiven Entfärbung einsetzen.

Ein dritter Gegenstand der vorliegenden Erfindung ist daher ein Verfahren zur Färbung und reduktiven Entfärbung von keratinischen Fasern, umfassend die folgenden Schritte in der angegebenen Reihenfolge

(I) Applizierung eines kosmetischen Färbemittels, das mindestens einen direktziehenden Farbstoff und/oder mindestens ein Oxlidationsfarbstoffvorprodukt enthält, auf die keratinischen Fasern,
(II) Einwirkenlassen des Färbemittels für einen Zeitraum von 5 bis 60 Minuten,
(III) Ausspülen des Färbemittels,
(IV) Herstellung eines anwendungsbereiten Entfärbemittels durch Vermischen eines Mittels des ersten Erfindungsgegenstands mit einem Mittel, dass mindestens eine organische und/oder anorganische Säure aus der Gruppe Zitronensäure, Weinsäure, Äpfelsäure, Milchsäure, Essigsäure, Schwefelsäure, Salzsäure, Phosphorsäure, Methansulfonsäure, Benzoesäure und/oder 1-Hydroxyethan-1,1-diphosphonsäure enthält,
(V) Applizierung des (in Schritt (IV) erhaltenen) anwendungsbereiten Entfärbemittels auf die keratinischen Fasern

(VI) Einwirkenlassen des Entfärbemittels für einen Zeitraum von 5 bis 60 Minuten, bevorzugt von 10 bis 55 Minuten, weiter bevorzugt von 15 bis 55 Minuten und besonders bevorzugt von 20 bis 50 Minuten,
(VII) Ausspülen des Entfärbemittels,
(VIII) gegebenenfalls Applizierung eines Nachbehandlungsmittels auf die keratinischen Fasern, wobei das Nachbehandlungsmittel mindestens ein Tensid enthält.

Die Schritte (I), (II) und (III) des Verfahrens stellen den Färbevorgang der Keratinfasern dar und werden demzufolge in direktzer zeitlicher Abfolge hintereinander ausgeführt. Für die Abfolge der Schritte (III) und (IV) besteht prinzipiell keine zeitliche Limitierung. So kann Schritt (IV) Stunden, Tage oder auch beispielsweise bis zu zwei Wochen nach Beendigung des Schrittes (III) erfolgen.

Das Verfahren soll jedoch das unerwünschte Farbergenis der Färbevorgangs der Schritte (I) bis (III) entfernen, daher versteht es sich von selbst, dass die Entfärbung nur dann vorgenommen werden kann, wenn die gefärbten Fasern auch noch das unerwünschtes Farbergebnis zeigen. Wurden die Keratinfasern beispielsweise mit direktziehenden Farbstoffen gefärbt und hat sich diese Färbung nach 2 Wochen schon komplett ausgewaschen, so ist ein im Anschluss daran stattfindender Entfärbevorgang weder notwendig noch erfindungsgemäß.

In Schritt (IV) des erfindungsgemäßen Verfahrens wird ein anwendungsbereites Entfärbemittel durch Vermischen eines Mittels des ersten Erfindungsgegenstand durch Vermischen mit einem weiteren Mittel, welches mindestens eine der vorgenannten Säuren enthält, herstellt. Die Schritte (IV), (V), (VI) und (VII) des Verfahrens stellen den Entfärbevorgang der Keratinsfasern dar und werden demzufolge wieder in direktzer zeitlicher Abfolge hintereinander ausgeführt.

Schritt (VIII) des Verfahrens, d.h. die Applizierung eines Nachbehandlungsmittels, ist optional. Für die Abfolge der Schritte (VII) und des optionalen Schrittes (VIII) besteht wieder keine zeitliche Limitierung.

Es ist jedoch von Vorteil, wenn die Nachbehandlung des Schrittes (VIII) maximal zwei Tage nach Abschluss des Schrittes (VII) erfolgt. Der Nachbehandlungsschritt (VIII) kann auch öfters als einmal wiederholt werden, beispielsweise wenn es sich bei dem Nachbehandlungsmittel um ein Shampoo handelt.

Im Zuge der zu dieser Erfindung führenden Arbeiten hat sich herausgestellt, dass eine Nachbehandlung in Schritt (VIII) mit einem Nachbehandlungsmittel, welches ein oder mehrere zwitterionische Tenside enthält, die als Struktureinheiten jeweils mindestens eine quartäre Ammoniumgruppe und eine Gruppierung -SO $^-$ besitzen, von ganz besonderem Vorteil ist.

Wenn das in Schritt (VIII) applizierte Nachbehandlungsmittel genau wie das Entfärbemittel ein erfindungsgemäßes zwitterionisches Tensid (b) enthält, ist die Inhibierung der Rückoxidation, d.h. die Verhinderung der Nachdunklung so lang anhaltend, dass diese sogar über mehrere Haarwäschen Bestand hat. Dies ist insbesondere der Fall, wenn es sich bei dem in Schritt (VIII) applizierten Nachbehandlungmittel um ein Shampoo handelt, das regelmäßig nach der Entfärbung angewendet wird.

Ganz besonders bevorzugt ist daher auch ein Verfahren zur Färbung und reduktiven Entfärbung von keratinischen Fasern, umfassend die folgenden Schritte in der angegebenen Reihenfolge

(I) Applizierung eines kosmetischen Färbemittels, das mindestens einen direktziehenden Farbstoff und/oder mindestens ein Oxlidationsfarbstoffvorprodukt enthält, auf die keratinischen Fasern,
(II) Einwirkenlassen des Färbemittels für einen Zeitraum von 5 bis 60 Minuten,
(III) Ausspülen des Färbemittels,
(IV) Herstellung eines anwendungsbereiten Entfärbemittels durch Vermischen eines Mittels des ersten Erfindungsgegenstands mit einem Mittel, dass mindestens eine organische und/oder anorganische Säure aus der Gruppe Zitronensäure, Weinsäure, Äpfelsäure, Milchsäure, Essigsäure, Schwefelsäure, Salzsäure, Phosphorsäure, Methansulfonsäure, Benzoesäure und/oder 1-Hydroxyethan-1,1-diphosphonsäure enthält,
(V) Applizierung des anwendungsbereiten Entfärbemittels aus Schritt (IV) auf die keratinischen Fasern
(VI) Einwirkenlassen des Entfärbemittels für einen Zeitraum von 5 bis 60 Minuten, bevorzugt von 10 bis 55 Minuten, weiter bevorzugt von 15 bis 55 Minuten und besonders bevorzugt von 20 bis 50 Minuten,
(VII) Ausspülen des Entfärbemittels,
(VIII) Applizierung eines Nachbehandlungsmittels auf die keratinischen Fasern, wobei das Nachbehandlungsmittel ein oder mehrere zwitterionische Tenside enthält, die als Struktureinheiten jeweils mindestens eine quartäre Ammoniumgruppe und eine Gruppierung -$SO_3^-$ besitzen.

<u>Beispiele</u>

1.1. Färbung

Es wurden die folgenden Formulierungen hergestellt (alle Angaben in Gew.-%):

Färbecreme (F1)

| Rohstoff | Gew.% |
|---|---|
| Cetearylalkohol | 8,5 |
| C12-C18 Fettalkohole | 3,0 |
| Ceteareth-20 | 0,5 |
| Ceteareth-12 | 0,5 |
| Plantacare 1200 UP (Laurylglucoside, 50 - 53 %ige wässrige Lösung) | 2,0 |
| Natrium Laureth-6 Carboxylat (21 %ige wässrige Lösung) | 10,0 |
| Natriummyreth Sulfat (68 - 73 %ige wässrige Lösung) | 2,8 |
| Sodium acrylate, trimethylammoniopropylacrylamide chloride copolymer (19 - 21 %ige wässrige Lösung) | 3,8 |
| Kaliumhydroxid | 0,83 |
| p-Toluylendiamin, Sulfat | 2,25 |
| m-Aminophenol | 0,075 |
| 2-Amino-3-hyd roxypyridin | 0,12 |
| Resorcin | 0,62 |
| 4-Chlorresorcin | 0,26 |
| 3-Amino-2-methylamino-6-methoxypyridin | 0,04 |
| 1,3-Bis(2,4-diaminophenoxy)propan, Tetrahydrochlorid | 0,05 |
| Ammoniumsulfat | 0,1 |
| Natriumsulfit | 0,4 |
| Ascorbinsäure | 0,1 |
| 1-Hydroxyethan-1,1-diphosphonsäure (60 %ige wässrige Lösung) | 0,2 |
| Ammoniak (25 %ige wässrige Lösung) | 7,2 |
| Wasser | Ad 100 |

Oxidationsmittel (Ox)

| Rohstoff | Gew.% |
|---|---|
| Natriumbenzoat | 0,04 |
| Dipicolinsäure | 0,1 |
| Dinatriumpyrophosphat | 0,1 |
| Kaliumhydroxid | 0,09 |
| 1,2-Propylenglycol | 1,0 |
| 1-Hydroxyethan-1,1-diphosphonsäure (60 %ige wässrige Lösung) | 0,25 |
| Paraffinum Liquidum | 0,30 |
| Steartrimoniumchlorid | 0,39 |
| Cetearylalkohol | 3,4 |
| Ceteareth-20 | 1,0 |
| Wasserstoffperoxid (50 %ige wässrige Lösung) | 12,0 |

Die Farbcreme (F1) und das Oxidationsmittel (Ox) wurden im Mengenverhältnis 1:1 gemischt und auf Haarsträhnen (Kerling Euronaturhaar weiß) appliziert. Das Gewichtsverhältnis Anwendungsmischung : Haar betrug 4:1, die Einwirkzeit betrug 30 Minuten bei einer Temperatur von 32 Grad Celsius. Anschließend wurden die Strähnen mit Wasser gespült, getrocknet und mindestens 24 Stunden bei Raumtemperatur ruhen gelassen. Die Strähnen wurden in einem dunkelbraunen Farbton gefärbt.

1.2. Entfärbung

Es wurden die folgenden Entfärbemittel hergestellt (alle Angaben in Gew.-% Aktivsubstanz):

| Rohstoff | V1 (Vergleich) | E1 (Erfindung) |
|---|---|---|
| Natriumdithionit | 8,8 | 8,8 |
| Cocamidopropylsultain | --- | 2,5 |
| Natriumhydroxid | 0,5 | 0,5 |
| Xanthan | 1,5 | 1,5 |
| Propylengylcol | 6,0 | 6,0 |

Der pH-Wert der beiden Formulierungen V1 und E1 lag bei einem Wert zwischen 8 und 10.

Für die Anwendung wurde jedes der Entfärbemittel V1 und E1 durch Zusatz einer wässrigen Zitronensäure-Lösung auf einen pH-Wert von 4 bis 5 eingestellt.

Dieses anwendungsbereite Entfärbemittel wurde auf die unter Punkt 1.1 colorierten Haare aufgetragen und für 45 Minuten bei einer Temperatur von 30 °C einwirken gelassen. Danach wurden die Strähnen für 2 Minuten mit Wasser ausgepült und getocknet.

Die Färbung der Strähnen wurde in Abhängigkeit von der Zeit visuell beurteilt. Die Bewertung der Farbintensität erfolgte anhand der folgenden Skala:
0 - Strähne besitzt keine wahrnehmbare Färbung mehr (weiß-blond, wie die urpsüngliche Färbung des eingesetzten Kerling Euronaturhaar weiß)
1 - Strähne mit schwacher Farbintensität gefärbt
2 - Strähne mit mittlerer Farbintensität gefärbt
3 - Strähne mit starker Farbintensität gefärbt
4 - Färbung der Strähne wie direkt nach der Färbung, kein Entfärbe-Effekt

| | V1 (Vergleich) | E1 (Erfindung) |
|---|---|---|
| Färbung der Strähne während des Auswaschens des Entfärbemittels | 0 | 0 |
| Färbung der Strähne direkt nach dem Auswaschen | 2 | 1 |
| Färbung der Strähne nach 10 Minuten | 3 | 1 |
| Färbung der Strähne nach 60 Minuten | 3 | 1 |
| Färbung der Strähne nach 1 Tag | 3 | 2 |

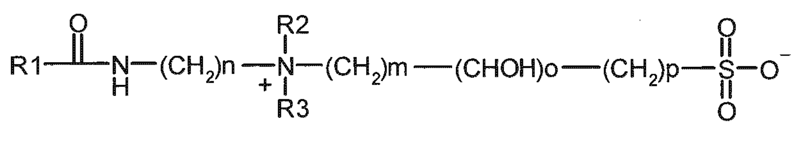
(I)

## Patentansprüche

1. Mittel zur reduktiven Entfärbung von gefärbten keratinischen Fasern, insbesondere menschlichen Haaren, enthaltend in einem wässrigen kosmetischen Träger

   (a) ein oder mehrere Reduktionsmittel aus der Gruppe Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat, Ammoniumthiosulfat, Hydroxymethansulfinsäure, Aminomethansulfinsäure, Cystein, Thiomilchsäure, Thioglycolsäure und/oder Ascorbinsäure, und
(b) ein oder mehrere zwitterionische Tenside, die als Struktureinheiten jeweils mindestens eine quartäre Ammoniumgruppe und mindestens eine Gruppierung $-SO_3^-$ besitzen, und
(c) ein oder mehrere Alkalisierungsmittel ausgewählt aus der Gruppe Natriumhydroxid, Kaliumhydroxid, Ammoniak, Monoethanolamin und/oder Arginin,

**dadurch gekennzeichnet, dass**

- die Gesamtmenge aller im Mittel enthaltenen direktziehenden Farbstoffe und Oxidationsfarbstoffvorprodukte bei einem Wert von maximal 0,2 Gew.-% - bezogen auf das Gesamtgewicht des Mittels - liegt, und
- das Gewichtsverhältnis aus der Gesamtmenge aller im Mittel enthaltenen Reduktionsmittel aus der Gruppe (a) zu der Gesamtmenge aller im Mittel enthaltenen zwitterionischen Tenside aus der Gruppe (b), d.h. das Gewichtsverhältnis (a)/(b), bei einem Wert von mindestens 2,6 und von höchstens 7,0 liegt.

**2.** Mittel nach Anspruch 1, **dadurch gekennzeichnet, dass** es

(a) ein oder mehrere Reduktionsmittel aus der Gruppe Natriumdithionit, Zinkdithionit, Kaliumdithionit, Natriumsulfit, Natriumhydrogensulfit, Kaliumsulfit, Kaliumhydrogensulfit, Ammoniumsulfit, Natriumthiosulfat, Kaliumthiosulfat und/oder Ammoniumthiosulfat in einer Gesamtmenge von 0,5 bis 20,5 Gew.-%, bevorzugt von 3,5 bis 15,5 Gew.-%, weiter bevorzugt von 6,0 bis 13,5 Gew.-% und besonders bevorzugt von 7,5 bis 11,5 Gew.-% - bezogen auf das Gesamtgewicht des Mittels - enthält.

**3.** Mittel nach einem der Ansprüche 1 bis 2, **dadurch gekennzeichnet, dass** es als zwitterionisches Tensid (b) ein oder mehrere Tenside der Formel (I) in einer Gesamtmenge von 0,1 bis 15,0 Gew.-%, bevorzugt von 0,5 bis 12,0 Gew.-%, weiter bevorzugt von 1,0 bis 9,0 Gew.-% und besonders bevorzugt von 2,0 bis 6,0 Gew.-% - bezogen auf das Gesamtgewicht des Mittels - enthält,

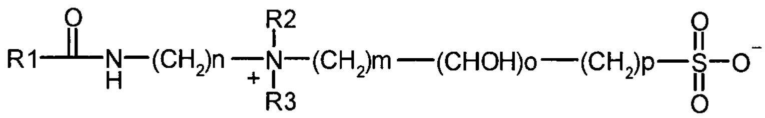
(I)

worin

R1 für eine lineare oder verzweigte $C_9$-$C_{29}$-Alkylgruppe, eine lineare oder verzweigte $C_9$-$C_{29}$-Alkenylgruppe oder eine lineare oder verzweigte Hydroxy-$C_9$-$C_{29}$-Alkylgruppe steht,
R2, R3 unabhängig voneinander für eine $C_1$-$C_6$-Alkylgruppe, eine $C_2$-$C_6$-Alkenylgruppe oder eine Hydroxy-$C_2$-$C_6$-alkylgruppe stehen,
n für eine ganze Zahl von 1 bis 6 steht,
m für eine ganze Zahl von 0 bis 6 steht,
o für eine ganze Zahl von 0 bis 6 steht,
p für eine ganze Zahl von 0 bis 6 steht,

mit der Maßgabe, dass die Summe aus m, o und p mindestens 1 beträgt.

**4.** Mittel nach einem der Ansprüche 1 bis 3, **dadurch gekennzeichnet, dass** das Gewichtsverhältnis aus der Gesamtmenge aller im Mittel enthaltenen Reduktionsmittel aus der Gruppe (a) zu der Gesamtmenge aller im Mittel enthaltenen zwitterionischen Tenside aus der Gruppe (b), d.h. das Gewichtsverhältnis (a)/(b), bei einem Wert von mindestens 3,2 liegt.

**5.** Mittel nach einem der Ansprüche 1 bis 4, **dadurch gekennzeichnet, dass** das Gewichtsverhältnis aus der Gesamtmenge aller im Mittel enthaltenen Reduktionsmittel aus der Gruppe (a) zu der Gesamtmenge aller im Mittel enthaltenen zwitterionischen Tenside aus der Gruppe (b), d.h. das Gewichtsverhältnis (a)/(b), bei einem Wert von höchstens 6,2, bevorzugt von höchstens 5,6 und besonders bevorzugt von höchstens 4,8 liegt.

6. Mittel nach einem der Ansprüche 1 bis 5, **dadurch gekennzeichnet, dass** es zusätzlich ein oder mehrere Polyole in einer Gesamtmenge von 0,5 bis 15,0 Gew.-%, bevorzugt von 2,5 bis 13,5 Gew.-%, weiter bevorzugt von 3,5 bis 11,5 Gew.-% und besonders bevorzugt von 4,5 bis 9,5 Gew.-% - bezogen auf das Gesamtgewicht des Mittels - enthält.

7. Mittel nach einem der Ansprüche 1 bis 6, **dadurch gekennzeichnet, dass** die Gesamtmenge aller im Mittel enthaltenen anionischen Tenside bei einem Wert von maximal 0,5 Gew.-%, bevorzugt von maximal 0,3 Gew.-%, weiter bevorzugt von maximal 0,1 Gew.-% und besonders bevorzugt von maximal 0,05 Gew.-% - bezogen auf das Gesamtgewicht des Mittels - liegt.

8. Mittel nach einem der Ansprüche 1 bis 7, **dadurch gekennzeichnet, dass** die Gesamtmenge aller im Mittel enthaltenen direktziehenden Farbstoffe und Oxidationsfarbstoffvorprodukte bei einem Wert von maximal 0,1 Gew.-%, bevorzugt von maximal 0,05 Gew.-% und besonders bevorzugt von maximal 0,01 Gew.-% - bezogen auf das Gesamtgewicht des Mittels - liegt.

9. Mittel nach einem der Ansprüche 1 bis 8, **dadurch gekennzeichnet, dass** es einen pH-Wert von 7,0 bis 12,0, bevorzugt von 7,5 bis 11,5, weiter bevorzugt von 8,0 bis 11,0 und ganz besonders bevorzugt von 8,5 bis 10,5 besitzt.

10. Mittel nach einem der Ansprüche 1 bis 9, **dadurch gekennzeichnet, dass** es zusätzlich ein oder mehrere Alkalisierungsmittel ausgewählt aus der Gruppe Natriumhydroxid und/oder Kaliumhydroxid, enthält.

11. Mittel nach einem der Ansprüche 1 bis 10, **dadurch gekennzeichnet, dass** es sich um eine klare, fließfähige Gelformulierung mit einem Transmissionsgrad T von mindestens 70 %, bevorzugt von mindestens 75 %, weiter bevorzugt von mindestens 80 % und besonders bevorzugt mindestens 85 % handelt, wobei sich der Transmissionsgrad T über die folgende Formel berechnet

$$T = \Phi ex/\Phi in$$

mit

$\phi ex$ gleich der Strahlungsintensität des aus dem Mittel austretenden, durchgelassenen Lichtstrahls und
$\phi in$ gleich der Strahlungsintensität des in das Mittel einfallenden Lichtstrahls.

12. Mehrkomponentenverpackungseinheit (Kit-of-parts) zur reduktiven Entfärbung von gefärbten keratinischen Fasern, welche getrennt voneinander konfektioniert

(I) in einem Container (I) ein Mittel (A) und
(II) in einem Container (II) ein Mittel (B) umfasst, wobei

- das Mittel (A) in Container (I) ein Mittel nach einem der Ansprüche 1 bis 11 ist und
- das Mittel (B) in Container (II) ein kosmetisches Mittel ist, das mindestens eine organische und/oder anorganische Säure aus der Gruppe Zitronensäure, Weinsäure, Äpfelsäure, Milchsäure, Essigsäure, Schwefelsäure, Salzsäure, Phosphorsäure, Methansulfonsäure, Benzoesäure Malonsäure, Oxalsäure und/oder 1-Hydroxyethan-1,1-diphosphonsäure enthält.

13. Mehrkomponentenverpackungseinheit (Kit-of-parts) nach Anspruch 12, wobei

- das Mittel (B) in Container (II) ein Mittel ist, das in einem wässrigen kosmetischen Träger mindestens eine organische Säure aus der Gruppe Zitronensäure, Weinsäure, Äpfelsäure, Milchsäure, Methansulfonsäure, Malonsäure, Oxalsäure und/oder 1-Hydroxyethan-1,1-diphosphonsäure enthält und einen pH-Wert von 1 bis 6, bevorzugt von 1,3 bis 4,5, weiter bevorzugt von 1,6 bis 4,0 und besonders bevorzugt von 2,0 bis 3,6 besitzt.

14. Verfahren zur Färbung und reduktiven Entfärbung von keratinischen Fasern, umfassend die folgenden Schritte in der angegebenen Reihenfolge

(I) Applizierung eines kosmetischen Färbemittels, das mindestens einen direktziehenden Farbstoff und/oder mindestens ein Oxlidationsfarbstoffvorprodukt enthält, auf die keratinischen Fasern
(II) Einwirkenlassen des Färbemittels für einen Zeitraum von 5 bis 60 Minuten

(III) Ausspülen des Färbemittels
(IV) Herstellung eines anwendungsbereiten Entfärbemittels durch Vermischen eines Mittels nach einem der Ansprüche 1 bis 11 mit einem Mittel, dass mindestens eine organische und/oder anorganische Säure aus der Gruppe Zitronensäure, Weinsäure, Äpfelsäure, Milchsäure, Essigsäure, Schwefelsäure, Salzsäure, Phosphorsäure, Methansulfonsäure, Benzoesäure und/oder 1-Hydroxyethan-1,1-diphosphonsäure enthält,
(V) Applizierung des anwendungsbereiten Entfärbemittels auf die keratinischen Fasern
(VI) Einwirkenlassen des Entfärbemittels für einen Zeitraum von 5 bis 60 Minuten, bevorzugt von 10 bis 55 Minuten, weiter bevorzugt von 15 bis 55 Minuten und besonders bevorzugt von 20 bis 50 Minuten,
(VII) Ausspülen des Entfärbemittels,
(VIII) gegebenenfalls Applizierung eines Nachbehandlungsmittels auf die keratinischen Fasern, wobei das Nachbehandlungsmittel mindestens ein amphoteres und/oder zwitterionisches Tensid enthält.

## Claims

**1.** An agent for reductively decolorizing dyed keratin fibers, in particular human hair, containing, in an aqueous cosmetic carrier,

(a) one or more reducing agents from the group comprising sodium dithionite, zinc dithionite, potassium dithionite, sodium sulfite, sodium hydrogen sulfite, potassium sulfite, potassium hydrogen sulfite, ammonium sulfite, sodium thiosulfate, potassium thiosulfate, ammonium thiosulfate, hydroxymethanesulfinic acid, aminomethanesulfinic acid, cysteine, thiolactic acid, thioglycolic acid and/or ascorbic acid, and
(b) one or more zwitterionic surfactants which each have, as structural units, at least one quaternary ammonium group and at least one $-SO_3^-$ grouping, and
(c) one or more alkalizing agents selected from the group comprising sodium hydroxide, potassium hydroxide, ammonia, monoethanolamine and/or arginine,

**characterized in that**

- the total amount of all direct dyes and oxidation dye precursors contained in the agent has a value of at most 0.2 wt.% based on the total weight of the agent, and
- the weight ratio of the total amount of all reducing agents of group (a) contained in the agent to the total amount of all zwitterionic surfactants of group (b) contained in the agent, i.e. the weight ratio (a)/(b), has a value of at least 2.6 and at most 7.0.

**2.** The agent according to claim 1, **characterized in that**

(a) it contains one or more reducing agents from the group comprising sodium dithionite, zinc dithionite, potassium dithionite, sodium sulfite, sodium hydrogen sulfite, potassium sulfite, potassium hydrogen sulfite, ammonium sulfite, sodium thiosulfate, potassium thiosulfate and/or ammonium thiosulfate in a total amount of from 0.5 to 20.5 wt.%, preferably 3.5 to 15.5 wt.%, more preferably 6.0 to 13.5 wt.%, and particularly preferably 7.5 to 11.5 wt.%, based on the total weight of the agent.

**3.** The agent according to one of claims 1 to 2, **characterized in that** it contains, as the zwitterionic surfactant (b), one or more surfactants of formula (I) in a total amount of from 0.1 to 15.0 wt.%, preferably 0.5 to 12.0 wt.%, more preferably 1.0 to 9.0 wt.%, and particularly preferably 2.0 to 6.0 wt.%, based on the total weight of the agent,

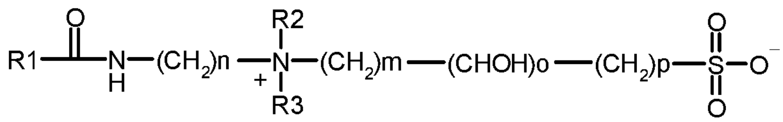

where

R1 represents a linear or branched $C_9$-$C_{29}$ alkyl group, a linear or branched $C_9$-$C_{29}$ alkenyl group or a linear or branched hydroxy-$C_9$-$C_{29}$ alkyl group,
R2, R3 represent, independently of one another, a $C_1$-$C_6$ alkyl group, a $C_2$-$C_6$ alkenyl group or a hydroxy-$C_2$-$C_6$ alkyl group,

n represents an integer from 1 to 6,
m represents an integer from 0 to 6,
o represents an integer from 0 to 6,
p represents an integer from 0 to 6,

with the proviso that the sum of m, o und p is at least 1.

**4.** The agent according to one of claims 1 to 3, **characterized in that** the weight ratio of the total amount of all reducing agents of group (a) contained in the agent to the total amount of all zwitterionic surfactants of group (b) contained in the agent, i.e. the weight ratio (a)/(b), has a value of at least 3.2.

**5.** The agent according to one of claims 1 to 4, **characterized in that** the weight ratio of the total amount of all reducing agents of group (a) contained in the agent to the total amount of all zwitterionic surfactants of group (b) contained in the agent, i.e. the weight ratio (a)/(b), has a value of at most 6.2, preferably at most 5.6, and particularly preferably at most 4.8.

**6.** The agent according to one of claims 1 to 5, **characterized in that** it additionally contains one or more polyols in a total amount of from 0.5 to 15.0 wt.%, preferably 2.5 to 13.5 wt.%, more preferably 3.5 to 11.5 wt.%, and particularly preferably 4.5 to 9.5 wt.%, based on the total weight of the agent.

**7.** The agent according to one of claims 1 to 6, **characterized in that** the total amount of all anionic surfactants contained in the agent has a value of at most 0.5 wt.%, preferably at most 0.3 wt.%, more preferably at most 0.1 wt.%, and particularly preferably at most 0.05 wt.%, based on the total weight of the agent.

**8.** The agent according to one of claims 1 to 7, **characterized in that** the total amount of all direct dyes and oxidation dye precursors contained in the agent has a value of at most 0.1 wt.%, preferably at most 0.05 wt.%, and particularly preferably at most 0.01 wt.%, based on the total weight of the agent.

**9.** The agent according to one of claims 1 to 8, **characterized in that** it has a pH of from 7.0 to 12.0, preferably 7.5 to 11.5, more preferably 8.0 to 11.0, and very particularly preferably 8.5 to 10.5.

**10.** The agent according to one of claims 1 to 9, **characterized in that** it additionally contains one or more alkalizing agents selected from the group comprising sodium hydroxide and/or potassium hydroxide.

**11.** The agent according to one of claims 1 to 10, **characterized in that** it is a clear, flowable gel formulation having a transmittance T of at least 70%, preferably at least 75%, more preferably at least 80%, and particularly preferably at least 85%, the transmittance T being calculated by the following formula:

$$T = \Phi ex/\Phi in$$

where

$\phi$ex is equal to the radiation intensity of the transmitted light beam emergent from the agent and
$\phi$in is equal to the radiation intensity of the light beam incident in the agent.

**12.** A multi-component packaging unit (kit-of-parts) for reductively decolorizing dyed keratin fibers, which comprises, packaged separately from one another,

(I) an agent (A) in a container (I) and
(II) an agent (B) in a container (II), wherein

- the agent (A) in container (I) is an agent according to one of claims 1 to 11 and
- the agent (B) in container (II) is a cosmetic agent which contains at least one organic and/or inorganic acid from the group comprising citric acid, tartaric acid, malic acid, lactic acid, acetic acid, sulfuric acid, hydrochloric acid, phosphoric acid, methanesulfonic acid, benzoic acid, malonic acid, oxalic acid and/or 1-hydroxyethane-1,1-diphosphonic acid.

**13.** The multi-component packaging unit (kit-of-parts) according to claim 12, wherein

- the agent (B) in container (II) is an agent which contains, in an aqueous cosmetic carrier, at least one organic acid from the group comprising citric acid, tartaric acid, malic acid, lactic acid, methanesulfonic acid, malonic acid, oxalic acid and/or 1-hydroxyethane-1,1-diphosphonic acid and has a pH of from 1 to 6, preferably 1.3 to 4.5, more preferably 1.6 to 4.0, and particularly preferably 2.0 to 3.6.

**14.** A method for dyeing and reductively decolorizing keratin fibers, comprising the following steps in the stated sequence:

(I) applying a cosmetic coloring agent, which contains at least one direct dye and/or at least one oxidation dye precursor, to the keratin fibers;
(II) allowing the coloring agent to act for a period of 5 to 60 minutes;
(III) rinsing out the coloring agent;
(IV) preparing a ready-to-apply decolorizing agent by mixing an agent according to one of claims 1 to 11 with an agent which contains at least one organic and/or inorganic acid from the group comprising citric acid, tartaric acid, malic acid, lactic acid, acetic acid, sulfuric acid, hydrochloric acid, phosphoric acid, methanesulfonic acid, benzoic acid and/or 1-hydroxyethane-1,1-diphosphonic acid;
(V) applying the ready-to-apply decolorizing agent to the keratin fibers;
(VI) allowing the decolorizing agent to act for a period of 5 to 60 minutes, preferably 10 to 55 minutes, more preferably 15 to 55 minutes, and particularly preferably 20 to 50 minutes;
(VII) rinsing out the decolorizing agent;
(VIII) optionally applying a post-treatment agent to the keratin fibers, wherein the post-treatment agent contains at least one amphoteric and/or zwitterionic surfactant.

## Revendications

**1.** Agent permettant la décoloration réductrice de fibres kératiniques teintes, en particulier de cheveux humains, contenu dans un support cosmétique aqueux

(a) un ou plusieurs agents de réduction du groupe constitué par le dithionite de sodium, le dithionite de zinc, le dithionite de potassium, le sulfite de sodium, l'hydrogénosulfite de sodium, le sulfite de potassium, l'hydrogénosulfite de potassium, le sulfite d'ammonium, le thiosulfate de sodium, le thiosulfate de potassium, le thiosulfate d'ammonium, l'acide hydroxyméthanesulfinique, l'acide aminométhanesulfinique, la cystéine, l'acide thiolactique, l'acide thioglycolique et/ou l'acide ascorbique, et
(b) un ou plusieurs tensioactifs zwitterioniques qui possèdent, comme unités de structure, respectivement au moins un groupe ammonium quaternaire et au moins un groupement $-SO_3^-$, et
(c) un ou plusieurs agents d'alcalisation choisis dans le groupe constitué par l'hydroxyde de sodium, l'hydroxyde de potassium, l'ammoniac, le monoéthanolamine et/ou l'arginine,

**caractérisé en ce que**

- la quantité totale de tous les colorants à action directe et précurseurs de colorant d'oxydation contenus dans l'agent est d'une valeur d'au plus 0,2 % en poids, sur la base du poids total de l'agent, et
- le rapport pondéral de la quantité totale de tous les agents de réduction du groupe (a) contenus dans l'agent à la quantité totale de tous les tensioactifs zwitterioniques du groupe (b) contenus dans l'agent, c'est-à-dire le rapport pondéral (a)/(b), est d'une valeur d'au moins 2,6 et d'au plus 7,0.

**2.** Agent selon la revendication 1, **caractérisé en ce qu'**il contient

(a) un ou plusieurs agents de réduction du groupe constitué par le dithionite de sodium, le dithionite de zinc, le dithionite de potassium, le sulfite de sodium, l'hydrogénosulfite de sodium, le sulfite de potassium, l'hydrogénosulfite de potassium, le sulfite d'ammonium, le thiosulfate de sodium, le thiosulfate de potassium et/ou le thiosulfate d'ammonium en une quantité totale de 0,5 à 20,5 % en poids, de préférence de 3,5 à 15,5 % en poids, plus préférablement de 6,0 à 13,5 % en poids et de manière particulièrement préférée de 7,5 à 11,5 % en poids, par rapport au poids total de l'agent.

**3.** Agent selon l'une des revendications 1 à 2, **caractérisé en ce qu'**il contient, comme tensioactif zwitterionique (b),

un ou plusieurs tensioactifs de formule (I) en une quantité totale de 0,1 à 15,0 % en poids, de préférence de 0,5 à 12,0 % en poids, plus préférablement de 1,0 à 9,0 % en poids et de manière particulièrement préférée de 2,0 à 6,0 % en poids, par rapport au poids total de l'agent,

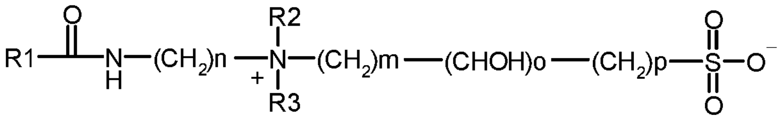

où

R1 représente un groupe alkyle en $C_9$-$C_{29}$ linéaire ou ramifié, un groupe alcényle en $C_9$-$C_{29}$ linéaire ou ramifié ou un groupe hydroxyalkyle en $C_9$-$C_{29}$ linéaire ou ramifié,
R2, R3 représentent indépendamment l'un de l'autre un groupe alkyle en $C_1$-$C_6$, un groupe alcényle en $C_2$-$C_6$ ou un groupe hydroxyalkyle en $C_2$-$C_6$,
n représente un nombre entier de 1 à 6,
m représente un nombre entier de 0 à 6,
o représente un nombre entier de 0 à 6,
p représente un nombre entier de 0 à 6,

à condition que la somme de m, o et p soit au moins 1.

**4.** Agent selon l'une des revendications 1 à 3, **caractérisé en ce que** le rapport pondéral de la quantité totale de tous les agents de réduction du groupe (a) contenus dans l'agent à la quantité totale de tous les tensioactifs zwitterioniques du groupe (b) contenus dans l'agent, c'est-à-dire le rapport pondéral (a)/(b), est d'une valeur d'au moins 3,2.

**5.** Agent selon l'une des revendications 1 à 4, **caractérisé en ce que** le rapport pondéral de la quantité totale de tous les agents de réduction du groupe (a) contenus dans l'agent à la quantité totale de tous les tensioactifs zwitterioniques du groupe (b) contenus dans l'agent, c'est-à-dire le rapport pondéral (a)/(b), est d'une valeur d'au plus 6,2, de préférence d'au plus 5,6 et de manière particulièrement préférée d'au plus 4,8.

**6.** Agent selon l'une des revendications 1 à 5, **caractérisé en ce qu'**il contient en outre un ou plusieurs polyols en une quantité totale de 0,5 à 15,0 % en poids, de préférence de 2,5 à 13,5 % en poids, plus préférablement de 3,5 à 11,5 % en poids et de manière particulièrement préférée de 4,5 à 9,5 % en poids, par rapport au poids total de l'agent.

**7.** Agent selon l'une des revendications 1 à 6, **caractérisé en ce que** la quantité totale de tous les tensioactifs anioniques contenus dans l'agent est d'une valeur d'au plus 0,5 % en poids, de préférence d'au plus 0,3 % en poids, plus préférablement d'au plus 0,1 % en poids et de manière particulièrement préférée d'au plus 0,05 % en poids, par rapport au poids total de l'agent.

**8.** Agent selon l'une des revendications 1 à 7, **caractérisé en ce que** la quantité totale de tous les colorants à action directe et précurseurs de colorant d'oxydation contenus dans l'agent est d'une valeur d'au plus 0,1 % en poids, de préférence d'au plus 0,05 % en poids et de manière particulièrement préférée d'au plus 0,01 % en poids, par rapport au poids total de l'agent.

**9.** Agent selon l'une des revendications 1 à 8, **caractérisé en ce qu'**il possède un pH de 7,0 à 12,0, de préférence de 7,5 à 11,5, plus préférablement de 8,0 à 11,0 et de manière tout particulièrement préférée de 8,5 à 10,5.

**10.** Agent selon l'une des revendications 1 à 9, **caractérisé en ce qu'**il contient en outre un ou plusieurs agents d'alcalisation choisis dans le groupe constitué par l'hydroxyde de sodium et/ou l'hydroxyde de potassium.

**11.** Agent selon l'une des revendications 1 à 10, **caractérisé en ce qu'**il s'agit d'une formulation de gel limpide et fluide avec une transmittance T d'au moins 70 %, de préférence d'au moins 75 %, plus préférablement d'au moins 80 % et de manière particulièrement préférée d'au moins 85 %, la transmittance T étant calculée à l'aide de la formule suivante

$$T = \Phi ex/\Phi in$$

avec

ϕex égal à l'intensité de rayonnement du faisceau lumineux transmis et émergeant de l'agent et
ϕin égal à l'intensité de rayonnement du faisceau lumineux entrant dans l'agent.

**12.** Unité d'emballage à multiples composants (kit d'éléments) permettant la décoloration réductrice de fibres kératiniques teintes, qui sont confectionnés séparément les uns des autres

(I) comprenant un agent (A) dans un récipient (I) et
(II) comprenant un agent (B) dans un récipient (II),

- l'agent (A) dans le récipient (I) étant un agent selon l'une des revendications 1 à 11 et
- l'agent (B) dans le récipient (II) étant un agent cosmétique qui contient au moins un acide organique et/ou inorganique du groupe constitué par l'acide citrique, l'acide tartrique, l'acide malique, l'acide lactique, l'acide acétique, l'acide sulfurique, l'acide chlorhydrique, l'acide phosphorique, l'acide méthanesulfonique, l'acide benzoïque, l'acide malonique, l'acide oxalique et/ou l'acide 1-hydroxyéthane-1,1-diphosphonique.

**13.** Unité d'emballage à plusieurs composants (kit d'éléments) selon la revendication 12, dans laquelle

- l'agent (B) dans le récipient (II) est un agent qui, dans un support cosmétique aqueux, contient au moins un acide organique du groupe constitué par l'acide citrique, l'acide tartrique, l'acide malique, l'acide lactique, l'acide méthanesulfonique, l'acide malonique, l'acide oxalique et/ou l'acide 1-hydroxyéthane-1,1-diphosphonique et possède un pH de 1 à 6, de préférence de 1,3 à 4,5, plus préférablement de 1,6 à 4,0 et de manière particulièrement préférée de 2,0 à 3,6.

**14.** Procédé permettant la coloration et la décoloration réductrice de fibres kératiniques, comprenant les étapes suivantes dans l'ordre indiqué

(I) appliquer un agent de coloration cosmétique contenant au moins un colorant à action directe et/ou au moins un précurseur de colorant d'oxydation sur les fibres kératiniques
(II) laisser agir l'agent de coloration pendant une durée de 5 à 60 minutes
(III) rincer l'agent de coloration
(IV) fabriquer un agent de décoloration prêt à l'emploi par mélange d'un agent selon l'une des revendications 1 à 11 avec un agent contenant au moins un acide organique et/ou inorganique du groupe constitué par l'acide citrique, l'acide tartrique, l'acide malique, l'acide lactique, l'acide acétique, l'acide sulfurique, l'acide chlorhydrique, l'acide phosphorique, l'acide méthanesulfonique, l'acide benzoïque et/ou l'acide 1-hydroxyéthane-1,1-diphosphonique,
(V) appliquer l'agent de décoloration prêt à l'emploi sur les fibres kératiniques
(VI) laisser agir l'agent de décoloration pendant une durée de 5 à 60 minutes, de préférence de 10 à 55 minutes, plus préférablement de 15 à 55 minutes et de manière particulièrement préférée de 20 à 50 minutes,
(VII) rincer l'agent de décoloration,
(VIII) éventuellement, appliquer un agent de post-traitement sur les fibres kératiniques, l'agent de post-traitement contenant au moins un tensioactif amphotère et/ou zwitterionique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1300136 A2
- WO 2008055756 A2


### In der Beschreibung aufgeführte Nicht-Patentliteratur


- **KH. SCHRADER.** Grundlagen und Rezepturen der Kosmetika. Hüthig Buch Verlag, 1989
- *CHEMICAL ABSTRACTS,* 7775-14-6
- *CHEMICAL ABSTRACTS,* 7779-86-4
- *CHEMICAL ABSTRACTS,* 14293-73-3
- *CHEMICAL ABSTRACTS,* 7757-83-7
- *CHEMICAL ABSTRACTS,* 7631-90-5
- *CHEMICAL ABSTRACTS,* 10117-38-1
- *CHEMICAL ABSTRACTS,* 7773-03-7
- *CHEMICAL ABSTRACTS,* 10196-04-0
- *CHEMICAL ABSTRACTS,* 7772-98-7
- *CHEMICAL ABSTRACTS,* 10294-66-3
- *CHEMICAL ABSTRACTS,* 7783-18-8
- *CHEMICAL ABSTRACTS,* 79-25-4
- *CHEMICAL ABSTRACTS,* 118201-33-5
- *CHEMICAL ABSTRACTS,* 68-11-1
- *CHEMICAL ABSTRACTS,* 50-81-7